# EUROPEAN PATENT SPECIFICATION

(11) **EP 3 498 854 B1**
(45) Date of publication and mention of the grant of the patent: **22.04.2020**
(21) Application number: 18211993.3
(22) Date of filing: 12.12.2018
(51) Int. Cl.: C12P 13/08, C12R 1/15

(54) **METHOD FOR THE FERMENTATIVE PRODUCTION OF L-LYSINE**
VERFAHREN ZUR FERMENTATIVEN HERSTELLUNG VON L-LYSIN
PROCÉDÉ DE PRODUCTION DE L-LYSINE PAR FERMENTATION

(30) Priority: 14.12.2017 EP 17207318
(43) Date of publication of application: 19.06.2019
(73) Proprietor: Evonik Operations GmbH, 45128 Essen (DE)
(72) Inventor: Voß, Kornelia, 33803 Steinhagen (DE); Thierbach, Georg, 33613 Bielefeld (DE); Bekel, Thomas, 33790 Halle (Westf.) (DE)
(74) Representative: Evonik Patent Association

(56) References cited:
- WO-A2-02/086137
- US-A1- 2002 028 490
- US-A1- 2006 166 338
- MASATO IKEDA ET AL: "A genome-based approach to create a minimally mutated Corynebacterium glutamicum strain for efficient l-lysine production", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 33, no. 7, 28 February 2006 (2006-02-28), pages 610-615, XP019388133, ISSN: 1476-5535, DOI: 10.1007/S10295-006-0104-5

### SEQUENCE LISTING

<110> Evonik Degussa GmbH
<120> Method for the fermentative production of L-lysine
<130> filing number: 201700371
<160> 22
<170> PatentIn version 3.5
<210> 1
   <211> 3503
   <212> DNA
   <213> Corynebacterium glutamicum ATCC13032
<220>
   <221> misc_feature
   <222> (1)..(3503)
   <223> nucleotide sequence comprising locus_tag NCgl1926 disclosed in GenBank accession number NC_003450
<220>
   <221> misc_feature
   <222> (781)..(781)
   <223> nucleobase adenine
<220>
   <221> CDS
   <222> (1001)..(2500)
<220>
   <221> misc_feature
   <222> (1682)..(1684)
   <223> gtc codon for valine at position 228
<220>
   <221> misc_feature
   <222> (1683)..(1683)
   <223> nucleobase thymine
<220>
   <221> misc_feature
   <222> (2228)..(2230)
   <223> ttc codon for phenylalanine at position 410
<220>
   <221> misc_feature
   <222> (2230)..(2230)
   <223> nucleobase cytosine
<220>
   <221> misc_feature
   <222> (2501)..(2503)
   <223> stop codon
<400> 1
   tccgtatgct cccaaacctc cgcctgggat gatggttgtg ggggagcgtt tgcccaacac        60
   cctcaccata ggggtgatca gcttgatgaa cagtggtgga tacatcatgt ccagccacgg       120
   actgttgagg accagcgctg gaatcttctc aatggcagct ggattgcttg tgcgcatctg      180
   ggacatccac agaggaacga tgagtccacc agtggaatgg gcgacgggga ctagctcagg      240
   gtgggtggaa gagatgacct cggcagcagc tgttaagtca gggaagtaat gggcaagatc      300
   agaggtgtag tgccactgct gtcctggacg gtaggagcgt ccacattttc taagatcaat      360
   gccgtacaca gcaaaaccgg cattgtggaa aaactccgcg aattcagtgt ggaagaagta      420
   gtccgtcatg ccgtgaaccc acagcaacgc tgggcgggca gcaaaagact cgtccgcgtg      480
   attgtctggg ttgtagcgca caaccgttgt cacaacatct gtttcattat ccgggtcgtc      540
   gccgagctcg atggtgaggt tttggtagcc ctctcccagg aagtctggtt tccactgctg      600
   catctgattc atacgtataa cgataggtcg attgttggtg tggtgtgcgc gagtcgactg      660
   aaatgttcac gtggtgaaac ttccgcgata ctactcatgt ttgcgaattg cacatttact      720
   aactttgcaa attgggggag gggtagcgcg gggggaggaa ttcgcatgag aaaggggaat      780
   atcccgtgct tgtttattca gctcgaggtg gcaggcgtac actctatatt cacggacaat      840
   gtgtacccac gctttcttgt aagaaacaag aagggtaacg ccccacgcgt cagtcaaaaa      900
   tatggccaac acttgcattc gggtgctggc gatcatttat gagatgacgc cttgtgttgg      960
   tgttcggcag agaactcgcg gagataaaag gaagttgaac atg tca gat tcc ccg      1015
                                              Met Ser Asp Ser Pro
                                              1               5
   aag aac gca ccg agg att acc gat gag gca gat gta gtt ctc att ggt      1063
   Lys Asn Ala Pro Arg Ile Thr Asp Glu Ala Asp Val Val Leu Ile Gly
                   10                  15                  20
   gcc ggt atc atg agc tcc acg ctg ggt gca atg ctg cgt cag ctg gag      1111
   Ala Gly Ile Met Ser Ser Thr Leu Gly Ala Met Leu Arg Gln Leu Glu
               25                  30                  35
   cca agc tgg act cag atc gtc ttc gag cgt ttg gat gga ccg gca caa      1159
   Pro Ser Trp Thr Gln Ile Val Phe Glu Arg Leu Asp Gly Pro Ala Gln
           40                  45                  50
   gag tcg tcc tcc ccg tgg aac aat gca gga acc ggc cac tct gct cta      1207
   Glu Ser Ser Ser Pro Trp Asn Asn Ala Gly Thr Gly His Ser Ala Leu
       55                  60                  65
   tgc gag ctg aac tac acc cca gag gtt aag ggc aag gtt gaa att gcc      1255
   Cys Glu Leu Asn Tyr Thr Pro Glu Val Lys Gly Lys Val Glu Ile Ala
   70                  75                  80                  85
   aag gct gta gga atc aac gag aag ttc cag gtt tcc cgt cag ttc tgg      1303
   Lys Ala Val Gly Ile Asn Glu Lys Phe Gln Val Ser Arg Gln Phe Trp
                   90                  95                  100
   tct cac ctc gtt gaa gag gga gtg ctg tct gat cct aag gaa ttc atc      1351
   Ser His Leu Val Glu Glu Gly Val Leu Ser Asp Pro Lys Glu Phe Ile
               105                 110                 115
   aac cct gtt cct cac gta tct ttc ggc cag ggc gca gat cag gtt gca      1399
   Asn Pro Val Pro His Val Ser Phe Gly Gln Gly Ala Asp Gln Val Ala
           120                 125                 130
   tac atc aag gct cgc tac gaa gct ttg aag gat cac cca ctc ttc cag        1447
   Tyr Ile Lys Ala Arg Tyr Glu Ala Leu Lys Asp His Pro Leu Phe Gln
       135                 140                 145
   ggc atg acc tac gct gac gat gaa gct acc ttc acc gag aag ctg cct        1495
   Gly Met Thr Tyr Ala Asp Asp Glu Ala Thr Phe Thr Glu Lys Leu Pro
   150                 155                 160                 165
   ttg atg gca aag ggc cgt gac ttc tct gat cca gta gca atc tct tgg        1543
   Leu Met Ala Lys Gly Arg Asp Phe Ser Asp Pro Val Ala Ile Ser Trp
                   170                 175                 180
   atc gat gaa ggc acc gac atc aac tac ggt gct cag acc aag cag tac        1591
   Ile Asp Glu Gly Thr Asp Ile Asn Tyr Gly Ala Gln Thr Lys Gln Tyr
               185                 190                 195
   ctg gat gca gct gaa gtt gaa ggc act gaa atc cgc tat ggc cac gaa        1639
   Leu Asp Ala Ala Glu Val Glu Gly Thr Glu Ile Arg Tyr Gly His Glu
           200                 205                 210
   gtc aag agc atc aag gct gat ggc gca aag tgg atc gtg acc gtc aag        1687
   Val Lys Ser Ile Lys Ala Asp Gly Ala Lys Trp Ile Val Thr Val Lys
       215                 220                 225
   aac gta cac act ggc gac acc aag acc atc aag gca aac ttc gtg ttc        1735
   Asn Val His Thr Gly Asp Thr Lys Thr Ile Lys Ala Asn Phe Val Phe
   230                 235                 240                 245
   gtc ggc gca ggc gga tac gca ctg gat ctg ctt cgc agc gca ggc atc        1783
   Val Gly Ala Gly Gly Tyr Ala Leu Asp Leu Leu Arg Ser Ala Gly Ile
                   250                 255                 260
   cca cag gtc aag ggc ttc gct gga ttc cca gta tcc ggc ctg tgg ctt        1831
   Pro Gln Val Lys Gly Phe Ala Gly Phe Pro Val Ser Gly Leu Trp Leu
               265                 270                 275
   cgt tgc acc aac gag gaa ctg atc gag cag cac gca gcc aag gta tat        1879
   Arg Cys Thr Asn Glu Glu Leu Ile Glu Gln His Ala Ala Lys Val Tyr
           280                 285                 290
   ggc aag gca tct gtt ggc gct cct cca atg tct gtt cct cac ctt gac        1927
   Gly Lys Ala Ser Val Gly Ala Pro Pro Met Ser Val Pro His Leu Asp
       295                 300                 305
   acc cgc gtt atc gag ggt gaa aag ggt ctg ctc ttt gga cct tac ggt        1975
   Thr Arg Val Ile Glu Gly Glu Lys Gly Leu Leu Phe Gly Pro Tyr Gly
   310                 315                 320                 325
   ggc tgg acc cct aag ttc ttg aag gaa ggc tcc tac ctg gac ctg ttc        2023
   Gly Trp Thr Pro Lys Phe Leu Lys Glu Gly Ser Tyr Leu Asp Leu Phe
                   330                 335                 340
   aag tcc atc cgc cca gac aac att cct tcc tac ctt ggc gtt gct gct        2071
   Lys Ser Ile Arg Pro Asp Asn Ile Pro Ser Tyr Leu Gly Val Ala Ala
               345                 350                 355
   cag gaa ttt gat ctg acc aag tac ctt gtc act gaa gtt ctc aag gac        2119
   Gln Glu Phe Asp Leu Thr Lys Tyr Leu Val Thr Glu Val Leu Lys Asp
           360                 365                 370
   cag gac aag cgt atg gat gct ctt cgc gag tac atg cca gag gca caa        2167
   Gln Asp Lys Arg Met Asp Ala Leu Arg Glu Tyr Met Pro Glu Ala Gln
       375                 380                 385
   aac ggc gat tgg gag acc atc gtt gcc gga cag cgt gtt cag gtt att        2215
   Asn Gly Asp Trp Glu Thr Ile Val Ala Gly Gln Arg Val Gln Val Ile
   390                 395                 400                 405
   aag cct gca gga ttc cct aag ttc ggt tcc ctg gaa ttc ggc acc acc        2263
   Lys Pro Ala Gly Phe Pro Lys Phe Gly Ser Leu Glu Phe Gly Thr Thr
                   410                 415                 420
   ttg atc aac aac tcc gaa ggc acc atc gcc gga ttg ctc ggt gct tcc        2311
   Leu Ile Asn Asn Ser Glu Gly Thr Ile Ala Gly Leu Leu Gly Ala Ser
               425                 430                 435
   cct gga gca tcc atc gca cct tcc gca atg atc gag ctg ctt gag cgt        2359
   Pro Gly Ala Ser Ile Ala Pro Ser Ala Met Ile Glu Leu Leu Glu Arg
           440                 445                 450
   tgc ttc ggt gac cgc atg atc gag tgg ggc gac aag ctg aag gac atg        2407
   Cys Phe Gly Asp Arg Met Ile Glu Trp Gly Asp Lys Leu Lys Asp Met
       455                 460                 465
   atc cct tcc tac ggc aag aag ctt gct tcc gag cca gca ctg ttt gag        2455
   Ile Pro Ser Tyr Gly Lys Lys Leu Ala Ser Glu Pro Ala Leu Phe Glu
   470                 475                 480                 485
   cag cag tgg gca cgc acc cag aag acc ctg aag ctt gag gaa gcc            2500
   Gln Gln Trp Ala Arg Thr Gln Lys Thr Leu Lys Leu Glu Glu Ala
                   490                 495                 500
   taaatcttct aactgctttc tttaaagcac ccgcacatgt ctgttgaggt ttcacctgcg     2560
   gagacaatct ccgccttcat gggttggaac tgacacagtt gaaggcatgt cgggtgcttt     2620
   gcgtattctt tgccagtgtg atttaggcga caccaataat ttattgggta tccaccaatt     2680
   accgctgtga gcactgcaaa ttacgtattc gaaaagccat gtccaccacg tgttctatcc     2740
   tggcggcatg caaaaaatca ccccaaacat ctggtgccaa ggcaccgcag acgaagcagc     2800
   cgaattctac gtcaatgcgt tttctgagtt tccgggtggc gcagaagtac tcaccacagt     2860
   taagtatccc gaagctggct tgctggactt ccaggagcct ttcgcaggaa aaaccttgac     2920
   ggtggaactc gctatctcag gctttaagat catcttgatc aatgctggtg aagagttcac     2980
   tcccaaccca tcgatcagct tcatggtgaa ttttgatgcg gtgcgtgatg aaaatgccaa     3040
   agagcacctt gatgcggtgt gggaaaaact ccatgaaggc ggcagcacac tgatgccagt     3100
   cgatacttac ccattttcgg aatactacgg gtgggtacaa gacaaatatg gtgtgagctg     3160
   gcaattgatg ctcagccgcc cagaagaaaa gccaggtccc gcagtaatcc caacgctctt     3220
   atttggtggg gcagctcaaa atcaggcagg cccagctcaa gaaaactacg ttgaggtgtt     3280
   cccgaactcc caacttggtg atcgtgcacc ttatggacag caaacaggtc ctgccactcc     3340
   tgaggccctc atgttttccc agttccaact cgacggtcag tggattttcg cgatggattc     3400
   cggagttgag caagatttca ccttcagtga gggtgtctca ttgatgtatg aagctcatgg     3460
   tcaagaagaa ctcgatgcca tctggaatgc actctcggca gtt                       3503
<210> 2
   <211> 500
   <212> PRT
   <213> Corynebacterium glutamicum ATCC13032
<400> 2
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ser Asp
               100                 105                 110
   Pro Lys Glu Phe Ile Asn Pro Val Pro His Val Ser Phe Gly Gln Gly
           115                 120                 125
   Ala Asp Gln Val Ala Tyr Ile Lys Ala Arg Tyr Glu Ala Leu Lys Asp
       130                 135                 140
   His Pro Leu Phe Gln Gly Met Thr Tyr Ala Asp Asp Glu Ala Thr Phe
   145                 150                 155                 160
   Thr Glu Lys Leu Pro Leu Met Ala Lys Gly Arg Asp Phe Ser Asp Pro
                   165                 170                 175
   Val Ala Ile Ser Trp Ile Asp Glu Gly Thr Asp Ile Asn Tyr Gly Ala
               180                 185                 190
   Gln Thr Lys Gln Tyr Leu Asp Ala Ala Glu Val Glu Gly Thr Glu Ile
           195                 200                 205
   Arg Tyr Gly His Glu Val Lys Ser Ile Lys Ala Asp Gly Ala Lys Trp
       210                 215                 220
   Ile Val Thr Val Lys Asn Val His Thr Gly Asp Thr Lys Thr Ile Lys
   225                 230                 235                 240
   Ala Asn Phe Val Phe Val Gly Ala Gly Gly Tyr Ala Leu Asp Leu Leu
                   245                 250                 255
   Arg Ser Ala Gly Ile Pro Gln Val Lys Gly Phe Ala Gly Phe Pro Val
               260                 265                 270
   Ser Gly Leu Trp Leu Arg Cys Thr Asn Glu Glu Leu Ile Glu Gln His
           275                 280                 285
   Ala Ala Lys Val Tyr Gly Lys Ala Ser Val Gly Ala Pro Pro Met Ser
       290                 295                 300
   Val Pro His Leu Asp Thr Arg Val Ile Glu Gly Glu Lys Gly Leu Leu
   305                 310                 315                 320
   Phe Gly Pro Tyr Gly Gly Trp Thr Pro Lys Phe Leu Lys Glu Gly Ser
                   325                 330                 335
   Tyr Leu Asp Leu Phe Lys Ser Ile Arg Pro Asp Asn Ile Pro Ser Tyr
               340                 345                 350
   Leu Gly Val Ala Ala Gln Glu Phe Asp Leu Thr Lys Tyr Leu Val Thr
           355                 360                 365
   Glu Val Leu Lys Asp Gln Asp Lys Arg Met Asp Ala Leu Arg Glu Tyr
       370                 375                 380
   Met Pro Glu Ala Gln Asn Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr Leu Lys
                   485                 490                 495
   Leu Glu Glu Ala
               500
<210> 3
   <211> 3503
   <212> DNA
   <213> Corynebacterium glutamicum
<220>
   <221> misc_feature
   <222> (781)..(781)
   <223> nucleobase adenine
<220>
   <221> CDS
   <222> (1001)..(2500)
<220>
   <221> misc_feature
   <222> (1682)..(1684)
   <223> gac codon for aspartic acid at position 228
<220>
   <221> mutation
   <222> (1683)..(1683)
   <223> nucleobase adenine
<220>
   <221> misc_feature
   <222> (2228)..(2230)
   <223> ttt codon for phenylalanine at position 410
<220>
   <221> misc_feature
   <222> (2230)..(2230)
   <223> nucleobase thymine
<220>
   <221> misc_feature
   <222> (2501)..(2503)
   <223> stop codon
<400> 3
   tccgtatgct cccaaacctc cgcctgggat gatggttgtg ggggagcgtt tgcccaacac      60
   cctcaccata ggggtgatca gcttgatgaa cagtggtgga tacatcatgt ccagccacgg     120
   actgttgagg accagcgctg gaatcttctc aatggcagct ggattgcttg tgcgcatctg     180
   ggacatccac agaggaacga tgagtccacc agtggaatgg gcgacgggga ctagctcagg     240
   gtgggtggaa gagatgacct cggcagcagc tgttaagtca gggaagtaat gggcaagatc     300
   agaggtgtag tgccactgct gtcctggacg gtaggagcgt ccacattttc taagatcaat     360
   gccgtacaca gcaaaaccgg cattgtggaa aaactccgcg aattcagtgt ggaagaagta      420
   gtccgtcatg ccgtgaaccc acagcaacgc tgggcgggca gcaaaagact cgtccgcgtg      480
   attgtctggg ttgtagcgca caaccgttgt cacaacatct gtttcattat ccgggtcgtc      540
   gccgagctcg atggtgaggt tttggtagcc ctctcccagg aagtctggtt tccactgctg      600
   catctgattc atacgtataa cgataggtcg attgttggtg tggtgtgcgc gagtcgactg      660
   aaatgttcac gtggtgaaac ttccgcgata ctactcatgt ttgcgaattg cacatttact      720
   aactttgcaa attgggggag ggggtagcgc gggggaggaa ttcgcatgag aaaggggaat      780
   atcccgtgct tgtttattca gctcgaggtg gcaggcgtac actctatatt cacggacaat      840
   gtgtacccac gctttcttgt aagaaacaag aagggtaacg ccccacgcgt cagtcaaaaa      900
   tatggccaac acttgcattc gggtgctggc gatcatttat gagatgacgc cttgtgttgg      960
   tgttcggcag agaactcgcg gagataaaag gaagttgaac atg tca gat tcc ccg       1015
                                              Met Ser Asp Ser Pro
                                              1               5
   aag aac gca ccg agg att acc gat gag gca gat gta gtt ctc att ggt       1063
   Lys Asn Ala Pro Arg Ile Thr Asp Glu Ala Asp Val Val Leu Ile Gly
                   10                  15                  20
   gcc ggt atc atg agc tcc acg ctg ggt gca atg ctg cgt cag ctg gag       1111
   Ala Gly Ile Met Ser Ser Thr Leu Gly Ala Met Leu Arg Gln Leu Glu
               25                  30                  35
   cca agc tgg act cag atc gtc ttc gag cgt ttg gat gga ccg gca caa       1159
   Pro Ser Trp Thr Gln Ile Val Phe Glu Arg Leu Asp Gly Pro Ala Gln
           40                  45                  50
   gag tcg tcc tcc ccg tgg aac aat gca gga acc ggc cac tct gct cta       1207
   Glu Ser Ser Ser Pro Trp Asn Asn Ala Gly Thr Gly His Ser Ala Leu
       55                  60                  65
   tgc gag ctg aac tac acc cca gag gtt aag ggc aag gtt gaa att gcc       1255
   Cys Glu Leu Asn Tyr Thr Pro Glu Val Lys Gly Lys Val Glu Ile Ala
   70                  75                  80                  85
   aag gct gta gga atc aac gag aag ttc cag gtt tcc cgt cag ttc tgg       1303
   Lys Ala Val Gly Ile Asn Glu Lys Phe Gln Val Ser Arg Gln Phe Trp
                   90                  95                  100
   tct cac ctc gtt gaa gag gga gtg ctg tct gat cct aag gaa ttc atc       1351
   Ser His Leu Val Glu Glu Gly Val Leu Ser Asp Pro Lys Glu Phe Ile
               105                 110                 115
   aac cct gtt cct cac gta tct ttc ggc cag ggc gca gat cag gtt gca       1399
   Asn Pro Val Pro His Val Ser Phe Gly Gln Gly Ala Asp Gln Val Ala
           120                 125                 130
   tac atc aag gct cgc tac gaa gct ttg aag gat cac cca ctc ttc cag       1447
   Tyr Ile Lys Ala Arg Tyr Glu Ala Leu Lys Asp His Pro Leu Phe Gln
       135                 140                 145
   ggc atg acc tac gct gac gat gaa gct acc ttc acc gag aag ctg cct       1495
   Gly Met Thr Tyr Ala Asp Asp Glu Ala Thr Phe Thr Glu Lys Leu Pro
   150                 155                 160                 165
   ttg atg gca aag ggc cgt gac ttc tct gat cca gta gca atc tct tgg        1543
   Leu Met Ala Lys Gly Arg Asp Phe Ser Asp Pro Val Ala Ile Ser Trp
                   170                 175                 180
   atc gat gaa ggc acc gac atc aac tac ggt gct cag acc aag cag tac        1591
   Ile Asp Glu Gly Thr Asp Ile Asn Tyr Gly Ala Gln Thr Lys Gln Tyr
               185                 190                 195
   ctg gat gca gct gaa gtt gaa ggc act gaa atc cgc tat ggc cac gaa        1639
   Leu Asp Ala Ala Glu Val Glu Gly Thr Glu Ile Arg Tyr Gly His Glu
           200                 205                 210
   gtc aag agc atc aag gct gat ggc gca aag tgg atc gtg acc gac aag        1687
   Val Lys Ser Ile Lys Ala Asp Gly Ala Lys Trp Ile Val Thr Asp Lys
       215                 220                 225
   aac gta cac act ggc gac acc aag acc atc aag gca aac ttc gtg ttc        1735
   Asn Val His Thr Gly Asp Thr Lys Thr Ile Lys Ala Asn Phe Val Phe
   230                 235                 240                 245
   gtc ggc gca ggc gga tac gca ctg gat ctg ctt cgc agc gca ggc atc        1783
   Val Gly Ala Gly Gly Tyr Ala Leu Asp Leu Leu Arg Ser Ala Gly Ile
                   250                 255                 260
   cca cag gtc aag ggc ttc gct gga ttc cca gta tcc ggc ctg tgg ctt        1831
   Pro Gln Val Lys Gly Phe Ala Gly Phe Pro Val Ser Gly Leu Trp Leu
               265                 270                 275
   cgt tgc acc aac gag gaa ctg atc gag cag cac gca gcc aag gta tat        1879
   Arg Cys Thr Asn Glu Glu Leu Ile Glu Gln His Ala Ala Lys Val Tyr
           280                 285                 290
   ggc aag gca tct gtt ggc gct cct cca atg tct gtt cct cac ctt gac        1927
   Gly Lys Ala Ser Val Gly Ala Pro Pro Met Ser Val Pro His Leu Asp
       295                 300                 305
   acc cgc gtt atc gag ggt gaa aag ggt ctg ctc ttt gga cct tac ggt        1975
   Thr Arg Val Ile Glu Gly Glu Lys Gly Leu Leu Phe Gly Pro Tyr Gly
   310                 315                 320                 325
   ggc tgg acc cct aag ttc ttg aag gaa ggc tcc tac ctg gac ctg ttc        2023
   Gly Trp Thr Pro Lys Phe Leu Lys Glu Gly Ser Tyr Leu Asp Leu Phe
                   330                 335                 340
   aag tcc atc cgc cca gac aac att cct tcc tac ctt ggc gtt gct gct        2071
   Lys Ser Ile Arg Pro Asp Asn Ile Pro Ser Tyr Leu Gly Val Ala Ala
               345                 350                 355
   cag gaa ttt gat ctg acc aag tac ctt gtc act gaa gtt ctc aag gac        2119
   Gln Glu Phe Asp Leu Thr Lys Tyr Leu Val Thr Glu Val Leu Lys Asp
           360                 365                 370
   cag gac aag cgt atg gat gct ctt cgc gag tac atg cca gag gca caa        2167
   Gln Asp Lys Arg Met Asp Ala Leu Arg Glu Tyr Met Pro Glu Ala Gln
       375                 380                 385
   aac ggc gat tgg gag acc atc gtt gcc gga cag cgt gtt cag gtt att        2215
   Asn Gly Asp Trp Glu Thr Ile Val Ala Gly Gln Arg Val Gln Val Ile
   390                 395                 400                 405
   aag cct gca gga ttt cct aag ttc ggt tcc ctg gaa ttc ggc acc acc        2263
   Lys Pro Ala Gly Phe Pro Lys Phe Gly Ser Leu Glu Phe Gly Thr Thr
                   410                 415                 420
   ttg atc aac aac tcc gaa ggc acc atc gcc gga ttg ctc ggt gct tcc      2311
   Leu Ile Asn Asn Ser Glu Gly Thr Ile Ala Gly Leu Leu Gly Ala Ser
               425                 430                 435
   cct gga gca tcc atc gca cct tcc gca atg atc gag ctg ctt gag cgt      2359
   Pro Gly Ala Ser Ile Ala Pro Ser Ala Met Ile Glu Leu Leu Glu Arg
           440                 445                 450
   tgc ttc ggt gac cgc atg atc gag tgg ggc gac aag ctg aag gac atg      2407
   Cys Phe Gly Asp Arg Met Ile Glu Trp Gly Asp Lys Leu Lys Asp Met
       455                 460                 465
   atc cct tcc tac ggc aag aag ctt gct tcc gag cca gca ctg ttt gag      2455
   Ile Pro Ser Tyr Gly Lys Lys Leu Ala Ser Glu Pro Ala Leu Phe Glu
   470                 475                 480                 485
   cag cag tgg gca cgc acc cag aag acc ctg aag ctt gag gaa gcc          2500
   Gln Gln Trp Ala Arg Thr Gln Lys Thr Leu Lys Leu Glu Glu Ala
                   490                 495                 500
   taaatcttct aactgctttc tttaaagcac ccgcacatgt ctgttgaggt ttcacctgcg    2560
   gagacaatct ccgccttcat gggttggaac tgacacagtt gaaggcatgt cgggtgcttt    2620
   gcgtattctt tgccagtgtg atttaggcga caccaataat ttattgggta tccaccaatt    2680
   accgctgtga gcactgcaaa ttacgtattc gaaaagccat gtccaccacg tgttctatcc    2740
   tggcggcatg caaaaaatca ccccaaacat ctggtgccaa ggcaccgcag acgaagcagc    2800
   cgaattctac gtcaatgcgt tttctgagtt tccgggtggc gcagaagtac tcaccacagt    2860
   taagtatccc gaagctggct tgctggactt ccaggagcct ttcgcaggaa aaaccttgac    2920
   ggtggaactc gctatctcag gctttaagat catcttgatc aatgctggtg aagagttcac    2980
   tcccaaccca tcgatcagct tcatggtgaa ttttgatgcg gtgcgtgatg aaaatgccaa    3040
   agagcacctt gatgcggtgt gggaaaaact ccatgaaggc ggcagcacac tgatgccagt    3100
   cgatacttac ccattttcgg aatactacgg gtgggtacaa gacaaatatg gtgtgagctg    3160
   gcaattgatg ctcagccgcc cagaagaaaa gccaggtccc gcagtaatcc caacgctctt    3220
   atttggtggg gcagctcaaa atcaggcagg cccagctcaa gaaaactacg ttgaggtgtt    3280
   cccgaactcc caacttggtg atcgtgcacc ttatggacag caaacaggtc ctgccactcc    3340
   tgaggccctc atgttttccc agttccaact cgacggtcag tggattttcg cgatggattc    3400
   cggagttgag caagatttca ccttcagtga gggtgtctca ttgatgtatg aagctcatgg    3460
   tcaagaagaa ctcgatgcca tctggaatgc actctcggca gtt                      3503
<210> 4
   <211> 500
   <212> PRT
   <213> Corynebacterium glutamicum
<210> 5
   <211> 1266
   <212> DNA
   <213> Corynebacterium glutamicum ATCC13032
<220>
   <221> CDS
   <222> (1)..(1263)
<400> 5
   gtg gcc ctg gtc gta cag aaa tat ggc ggt tcc tcg ctt gag agt gcg        48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct        96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat       144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt       192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc       240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg       288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc       336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc       384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc       432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg       480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt       528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag       576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca     1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat     1248
   Leu His Glu Gln Phe Gln Leu Gly Gly Glu Asp Glu Ala Val Val Tyr
                   405                 410                 415
   gca ggc acc gga cgc taa                                             1266
   Ala Gly Thr Gly Arg
               420
<210> 6
   <211> 421
   <212> PRT
   <213> Corynebacterium glutamicum ATCC13032
<400> 6
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 7
   <211> 1603
   <212> DNA
   <213> Corynebacterium glutamicum
<220>
   <221> misc_feature
   <222> (1)..(801)
   <223> sequence upstream of the site of mutation (5'-flanking sequence)
<220>
   <221> CDS
   <222> (120)..(1601)
<220>
   <221> misc_feature
   <222> (801)..(803)
   <223> gac codon for aspartic acid at position 228
<220>
   <221> mutation
   <222> (802)..(802)
   <223> nucleobase adenine
<220>
   <221> misc_feature
   <222> (803)..(1603)
   <223> sequence downstream of the site of mutation (3'-flanking sequence)
<220>
   <221> misc_feature
   <222> (1347)..(1349)
   <223> ttt codon for phenyalanine at position 410
<220>
   <221> misc_feature
   <222> (1349)..(1349)
   <223> nucleobase thymine
<400> 7
   cccacgcgtc agtcaaaaat atggccaaca cttgcattcg ggtgctggcg atcatttatg      60
   agatgacgcc ttgtgttggt gttcggcaga gaactcgcgg agataaaagg aagttgaac      119
   atg tca gat tcc ccg aag aac gca ccg agg att acc gat gag gca gat       167
   Met Ser Asp Ser Pro Lys Asn Ala Pro Arg Ile Thr Asp Glu Ala Asp
   1               5                   10                  15
   gta gtt ctc att ggt gcc ggt atc atg agc tcc acg ctg ggt gca atg       215
   Val Val Leu Ile Gly Ala Gly Ile Met Ser Ser Thr Leu Gly Ala Met
               20                  25                  30
   ctg cgt cag ctg gag cca agc tgg act cag atc gtc ttc gag cgt ttg       263
   Leu Arg Gln Leu Glu Pro Ser Trp Thr Gln Ile Val Phe Glu Arg Leu
           35                  40                  45
   gat gga ccg gca caa gag tcg tcc tcc ccg tgg aac aat gca gga acc       311
   Asp Gly Pro Ala Gln Glu Ser Ser Ser Pro Trp Asn Asn Ala Gly Thr
       50                  55                  60
   ggc cac tct gct cta tgc gag ctg aac tac acc cca gag gtt aag ggc       359
   Gly His Ser Ala Leu Cys Glu Leu Asn Tyr Thr Pro Glu Val Lys Gly
   65                  70                  75                  80
   aag gtt gaa att gcc aag gct gta gga atc aac gag aag ttc cag gtt       407
   Lys Val Glu Ile Ala Lys Ala Val Gly Ile Asn Glu Lys Phe Gln Val
                   85                  90                  95
   tcc cgt cag ttc tgg tct cac ctc gtt gaa gag gga gtg ctg tct gat       455
   Ser Arg Gln Phe Trp Ser His Leu Val Glu Glu Gly Val Leu Ser Asp
               100                 105                 110
   cct aag gaa ttc atc aac cct gtt cct cac gta tct ttc ggc cag ggc       503
   Pro Lys Glu Phe Ile Asn Pro Val Pro His Val Ser Phe Gly Gln Gly
           115                 120                 125
   gca gat cag gtt gca tac atc aag gct cgc tac gaa gct ttg aag gat          551
   Ala Asp Gln Val Ala Tyr Ile Lys Ala Arg Tyr Glu Ala Leu Lys Asp
       130                 135                 140
   cac cca ctc ttc cag ggc atg acc tac gct gac gat gaa gct acc ttc          599
   His Pro Leu Phe Gln Gly Met Thr Tyr Ala Asp Asp Glu Ala Thr Phe
   145                 150                 155                 160
   acc gag aag ctg cct ttg atg gca aag ggc cgt gac ttc tct gat cca          647
   Thr Glu Lys Leu Pro Leu Met Ala Lys Gly Arg Asp Phe Ser Asp Pro
                   165                 170                 175
   gta gca atc tct tgg atc gat gaa ggc acc gac atc aac tac ggt gct          695
   Val Ala Ile Ser Trp Ile Asp Glu Gly Thr Asp Ile Asn Tyr Gly Ala
               180                 185                 190
   cag acc aag cag tac ctg gat gca gct gaa gtt gaa ggc act gaa atc          743
   Gln Thr Lys Gln Tyr Leu Asp Ala Ala Glu Val Glu Gly Thr Glu Ile
           195                 200                 205
   cgc tat ggc cac gaa gtc aag agc atc aag gct gat ggc gca aag tgg          791
   Arg Tyr Gly His Glu Val Lys Ser Ile Lys Ala Asp Gly Ala Lys Trp
       210                 215                 220
   atc gtg acc gac aag aac gta cac act ggc gac acc aag acc atc aag          839
   Ile Val Thr Asp Lys Asn Val His Thr Gly Asp Thr Lys Thr Ile Lys
   225                 230                 235                 240
   gca aac ttc gtg ttc gtc ggc gca ggc gga tac gca ctg gat ctg ctt          887
   Ala Asn Phe Val Phe Val Gly Ala Gly Gly Tyr Ala Leu Asp Leu Leu
                   245                 250                 255
   cgc agc gca ggc atc cca cag gtc aag ggc ttc gct gga ttc cca gta          935
   Arg Ser Ala Gly Ile Pro Gln Val Lys Gly Phe Ala Gly Phe Pro Val
               260                 265                 270
   tcc ggc ctg tgg ctt cgt tgc acc aac gag gaa ctg atc gag cag cac          983
   Ser Gly Leu Trp Leu Arg Cys Thr Asn Glu Glu Leu Ile Glu Gln His
           275                 280                 285
   gca gcc aag gta tat ggc aag gca tct gtt ggc gct cct cca atg tct         1031
   Ala Ala Lys Val Tyr Gly Lys Ala Ser Val Gly Ala Pro Pro Met Ser
       290                 295                 300
   gtt cct cac ctt gac acc cgc gtt atc gag ggt gaa aag ggt ctg ctc         1079
   Val Pro His Leu Asp Thr Arg Val Ile Glu Gly Glu Lys Gly Leu Leu
   305                 310                 315                 320
   ttt gga cct tac ggt ggc tgg acc cct aag ttc ttg aag gaa ggc tcc         1127
   Phe Gly Pro Tyr Gly Gly Trp Thr Pro Lys Phe Leu Lys Glu Gly Ser
                   325                 330                 335
   tac ctg gac ctg ttc aag tcc atc cgc cca gac aac att cct tcc tac         1175
   Tyr Leu Asp Leu Phe Lys Ser Ile Arg Pro Asp Asn Ile Pro Ser Tyr
               340                 345                 350
   ctt ggc gtt gct gct cag gaa ttt gat ctg acc aag tac ctt gtc act         1223
   Leu Gly Val Ala Ala Gln Glu Phe Asp Leu Thr Lys Tyr Leu Val Thr
           355                 360                 365
   gaa gtt ctc aag gac cag gac aag cgt atg gat gct ctt cgc gag tac         1271
   Glu Val Leu Lys Asp Gln Asp Lys Arg Met Asp Ala Leu Arg Glu Tyr
       370                 375                 380
   atg cca gag gca caa aac ggc gat tgg gag acc atc gtt gcc gga cag      1319
   Met Pro Glu Ala Gln Asn Gly Asp Trp Glu Thr Ile Val Ala Gly Gln
   385                 390                 395                 400
   cgt gtt cag gtt att aag cct gca gga ttt cct aag ttc ggt tcc ctg      1367
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga      1415
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc      1463
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac      1511
   Glu Leu Leu Glu Arg Cys Phe Gly Asp Arg Met Ile Glu Trp Gly Asp
       450                 455                 460
   aag ctg aag gac atg atc cct tcc tac ggc aag aag ctt gct tcc gag      1559
   Lys Leu Lys Asp Met Ile Pro Ser Tyr Gly Lys Lys Leu Ala Ser Glu
   465                 470                 475                 480
   cca gca ctg ttt gag cag cag tgg gca cgc acc cag aag acc ct           1603
   Pro Ala Leu Phe Glu Gln Gln Trp Ala Arg Thr Gln Lys Thr
                   485                 490
<210> 8
   <211> 494
   <212> PRT
   <213> Corynebacterium glutamicum
<400> 8
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ser Asp
               100                 105                 110
   Pro Lys Glu Phe Ile Asn Pro Val Pro His Val Ser Phe Gly Gln Gly
           115                 120                 125
   Ala Asp Gln Val Ala Tyr Ile Lys Ala Arg Tyr Glu Ala Leu Lys Asp
       130                 135                 140
   His Pro Leu Phe Gln Gly Met Thr Tyr Ala Asp Asp Glu Ala Thr Phe
   145                 150                 155                 160
   Thr Glu Lys Leu Pro Leu Met Ala Lys Gly Arg Asp Phe Ser Asp Pro
                   165                 170                 175
   Val Ala Ile Ser Trp Ile Asp Glu Gly Thr Asp Ile Asn Tyr Gly Ala
               180                 185                 190
   Gln Thr Lys Gln Tyr Leu Asp Ala Ala Glu Val Glu Gly Thr Glu Ile
           195                 200                 205
   Arg Tyr Gly His Glu Val Lys Ser Ile Lys Ala Asp Gly Ala Lys Trp
       210                 215                 220
   Ile Val Thr Asp Lys Asn Val His Thr Gly Asp Thr Lys Thr Ile Lys
   225                 230                 235                 240
   Ala Asn Phe Val Phe Val Gly Ala Gly Gly Tyr Ala Leu Asp Leu Leu
                   245                 250                 255
   Arg Ser Ala Gly Ile Pro Gln Val Lys Gly Phe Ala Gly Phe Pro Val
               260                 265                 270
   Ser Gly Leu Trp Leu Arg Cys Thr Asn Glu Glu Leu Ile Glu Gln His
           275                 280                 285
   Ala Ala Lys Val Tyr Gly Lys Ala Ser Val Gly Ala Pro Pro Met Ser
       290                 295                 300
   Val Pro His Leu Asp Thr Arg Val Ile Glu Gly Glu Lys Gly Leu Leu
   305                 310                 315                 320
   Phe Gly Pro Tyr Gly Gly Trp Thr Pro Lys Phe Leu Lys Glu Gly Ser
                   325                 330                 335
   Tyr Leu Asp Leu Phe Lys Ser Ile Arg Pro Asp Asn Ile Pro Ser Tyr
               340                 345                 350
   Leu Gly Val Ala Ala Gln Glu Phe Asp Leu Thr Lys Tyr Leu Val Thr
               355                 360                 365
   Glu Val Leu Lys Asp Gln Asp Lys Arg Met Asp Ala Leu Arg Glu Tyr
           370                 375                 380
   Met Pro Glu Ala Gln Asn Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
               435                 440                 445
   Glu Leu Leu Glu Arg Cys Phe Gly Asp Arg Met Ile Glu Trp Gly Asp
           450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr
                   485                 490
<210> 9
   <211> 34
   <212> DNA
   <213> artificial sequence
<220>
   <223> primer
<220>
   <221> misc_feature
   <222> (1)..(34)
   <223> primer 1f-mqo-pK18
<400> 9
   ggtacccggg gatcctccca cgcgtcagtc aaaa 34
<210> 10
   <211> 33
   <212> DNA
   <213> artificial sequence
<220>
   <223> primer
<220>
   <221> misc_feature
   <222> (1)..(33)
   <223> primer 1r-mqo-pK18
<400> 10
   gcctgcaggt cgactagggt cttctgggtg cgt 33
<210> 11
   <211> 1634
   <212> DNA
   <213> artificial sequence
<220>
   <223> amplificate from Corynebacterium glutamicum DM2463
<220>
   <221> misc_feature
   <222> (1)..(1634)
   <223> polynucleotide mqo'-V228D
<220>
   <221> misc_feature
   <222> (1)..(16)
   <223> 5'-overlap to pK18mobsacB-XbaI
<220>
   <221> misc_feature
   <222> (17)..(1619)
   <223> nucleotide sequence of SEQ ID NO:7
<220>
   <221> misc_feature
   <222> (17)..(1619)
   <223> nucleotide sequence of DM2463
<220>
   <221> misc_feature
   <222> (17)..(817)
   <223> sequence upstream of the site mutation (5'-flanking sequence)
<220>
   <221> CDS
   <222> (136)..(1617)
<220>
   <221> misc_feature
   <222> (817)..(819)
   <223> gac codon for aspartic acid at position 228
<220>
   <221> mutation
   <222> (818)..(818)
   <223> nucleobase adenine
<220>
   <221> misc_feature
   <222> (819)..(1619)
   <223> sequence downstream of the site of mutation (3'-flanking sequence)
<220>
   <221> misc_feature
   <222> (1363)..(1365)
   <223> ttt codon for phenylalanine at position 410
<220>
   <221> misc_feature
   <222> (1365)..(1365)
   <223> nucleobase thymine
<220>
   <221> misc_feature
   <222> (1620)..(1634)
   <223> 3'-overlap to pK18mobsacB-XbaI
<400> 11
   ggtacccggg gatcctccca cgcgtcagtc aaaaatatgg ccaacacttg cattcgggtg        60
   ctggcgatca tttatgagat gacgccttgt gttggtgttc ggcagagaac tcgcggagat       120
   aaaaggaagt tgaac atg tca gat tcc ccg aag aac gca ccg agg att acc       171
                    Met Ser Asp Ser Pro Lys Asn Ala Pro Arg Ile Thr
                    1               5                   10
   gat gag gca gat gta gtt ctc att ggt gcc ggt atc atg agc tcc acg        219
   Asp Glu Ala Asp Val Val Leu Ile Gly Ala Gly Ile Met Ser Ser Thr
           15                  20                  25
   ctg ggt gca atg ctg cgt cag ctg gag cca agc tgg act cag atc gtc        267
   Leu Gly Ala Met Leu Arg Gln Leu Glu Pro Ser Trp Thr Gln Ile Val
       30                  35                  40
   ttc gag cgt ttg gat gga ccg gca caa gag tcg tcc tcc ccg tgg aac        315
   Phe Glu Arg Leu Asp Gly Pro Ala Gln Glu Ser Ser Ser Pro Trp Asn
   45                  50                  55                  60
   aat gca gga acc ggc cac tct gct cta tgc gag ctg aac tac acc cca        363
   Asn Ala Gly Thr Gly His Ser Ala Leu Cys Glu Leu Asn Tyr Thr Pro
                   65                  70                  75
   gag gtt aag ggc aag gtt gaa att gcc aag gct gta gga atc aac gag        411
   Glu Val Lys Gly Lys Val Glu Ile Ala Lys Ala Val Gly Ile Asn Glu
               80                  85                  90
   aag ttc cag gtt tcc cgt cag ttc tgg tct cac ctc gtt gaa gag gga        459
   Lys Phe Gln Val Ser Arg Gln Phe Trp Ser His Leu Val Glu Glu Gly
           95                  100                 105
   gtg ctg tct gat cct aag gaa ttc atc aac cct gtt cct cac gta tct        507
   Val Leu Ser Asp Pro Lys Glu Phe Ile Asn Pro Val Pro His Val Ser
       110                 115                 120
   ttc ggc cag ggc gca gat cag gtt gca tac atc aag gct cgc tac gaa        555
   Phe Gly Gln Gly Ala Asp Gln Val Ala Tyr Ile Lys Ala Arg Tyr Glu
   125                 130                 135                 140
   gct ttg aag gat cac cca ctc ttc cag ggc atg acc tac gct gac gat        603
   Ala Leu Lys Asp His Pro Leu Phe Gln Gly Met Thr Tyr Ala Asp Asp
                   145                 150                 155
   gaa gct acc ttc acc gag aag ctg cct ttg atg gca aag ggc cgt gac        651
   Glu Ala Thr Phe Thr Glu Lys Leu Pro Leu Met Ala Lys Gly Arg Asp
               160                 165                 170
   ttc tct gat cca gta gca atc tct tgg atc gat gaa ggc acc gac atc          699
   Phe Ser Asp Pro Val Ala Ile Ser Trp Ile Asp Glu Gly Thr Asp Ile
           175                 180                 185
   aac tac ggt gct cag acc aag cag tac ctg gat gca gct gaa gtt gaa          747
   Asn Tyr Gly Ala Gln Thr Lys Gln Tyr Leu Asp Ala Ala Glu Val Glu
       190                 195                 200
   ggc act gaa atc cgc tat ggc cac gaa gtc aag agc atc aag gct gat          795
   Gly Thr Glu Ile Arg Tyr Gly His Glu Val Lys Ser Ile Lys Ala Asp
   205                 210                 215                 220
   ggc gca aag tgg atc gtg acc gac aag aac gta cac act ggc gac acc          843
   Gly Ala Lys Trp Ile Val Thr Asp Lys Asn Val His Thr Gly Asp Thr
                   225                 230                 235
   aag acc atc aag gca aac ttc gtg ttc gtc ggc gca ggc gga tac gca          891
   Lys Thr Ile Lys Ala Asn Phe Val Phe Val Gly Ala Gly Gly Tyr Ala
               240                 245                 250
   ctg gat ctg ctt cgc agc gca ggc atc cca cag gtc aag ggc ttc gct          939
   Leu Asp Leu Leu Arg Ser Ala Gly Ile Pro Gln Val Lys Gly Phe Ala
           255                 260                 265
   gga ttc cca gta tcc ggc ctg tgg ctt cgt tgc acc aac gag gaa ctg          987
   Gly Phe Pro Val Ser Gly Leu Trp Leu Arg Cys Thr Asn Glu Glu Leu
       270                 275                 280
   atc gag cag cac gca gcc aag gta tat ggc aag gca tct gtt ggc gct         1035
   Ile Glu Gln His Ala Ala Lys Val Tyr Gly Lys Ala Ser Val Gly Ala
   285                 290                 295                 300
   cct cca atg tct gtt cct cac ctt gac acc cgc gtt atc gag ggt gaa         1083
   Pro Pro Met Ser Val Pro His Leu Asp Thr Arg Val Ile Glu Gly Glu
                   305                 310                 315
   aag ggt ctg ctc ttt gga cct tac ggt ggc tgg acc cct aag ttc ttg         1131
   Lys Gly Leu Leu Phe Gly Pro Tyr Gly Gly Trp Thr Pro Lys Phe Leu
               320                 325                 330
   aag gaa ggc tcc tac ctg gac ctg ttc aag tcc atc cgc cca gac aac         1179
   Lys Glu Gly Ser Tyr Leu Asp Leu Phe Lys Ser Ile Arg Pro Asp Asn
           335                 340                 345
   att cct tcc tac ctt ggc gtt gct gct cag gaa ttt gat ctg acc aag         1227
   Ile Pro Ser Tyr Leu Gly Val Ala Ala Gln Glu Phe Asp Leu Thr Lys
       350                 355                 360
   tac ctt gtc act gaa gtt ctc aag gac cag gac aag cgt atg gat gct         1275
   Tyr Leu Val Thr Glu Val Leu Lys Asp Gln Asp Lys Arg Met Asp Ala
   365                 370                 375                 380
   ctt cgc gag tac atg cca gag gca caa aac ggc gat tgg gag acc atc         1323
   Leu Arg Glu Tyr Met Pro Glu Ala Gln Asn Gly Asp Trp Glu Thr Ile
                   385                 390                 395
   gtt gcc gga cag cgt gtt cag gtt att aag cct gca gga ttt cct aag         1371
   Val Ala Gly Gln Arg Val Gln Val Ile Lys Pro Ala Gly Phe Pro Lys
               400                 405                 410
   ttc ggt tcc ctg gaa ttc ggc acc acc ttg atc aac aac tcc gaa ggc         1419
   Phe Gly Ser Leu Glu Phe Gly Thr Thr Leu Ile Asn Asn Ser Glu Gly
           415                 420                 425
   acc atc gcc gga ttg ctc ggt gct tcc cct gga gca tcc atc gca cct      1467
   Thr Ile Ala Gly Leu Leu Gly Ala Ser Pro Gly Ala Ser Ile Ala Pro
       430                 435                 440
   tcc gca atg atc gag ctg ctt gag cgt tgc ttc ggt gac cgc atg atc      1515
   Ser Ala Met Ile Glu Leu Leu Glu Arg Cys Phe Gly Asp Arg Met Ile
   445                 450                 455                 460
   gag tgg ggc gac aag ctg aag gac atg atc cct tcc tac ggc aag aag      1563
   Glu Trp Gly Asp Lys Leu Lys Asp Met Ile Pro Ser Tyr Gly Lys Lys
                   465                 470                 475
   ctt gct tcc gag cca gca ctg ttt gag cag cag tgg gca cgc acc cag      1611
   Leu Ala Ser Glu Pro Ala Leu Phe Glu Gln Gln Trp Ala Arg Thr Gln
               480                 485                 490
   aag acc ctagtcgacc tgcaggc                                           1634
   Lys Thr
<210> 12
   <211> 494
   <212> PRT
   <213> artificial sequence
<220>
   <223> Synthetic Construct
<400> 12
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ser Asp
               100                 105                 110
   Pro Lys Glu Phe Ile Asn Pro Val Pro His Val Ser Phe Gly Gln Gly
   115 120 125
   Ala Asp Gln Val Ala Tyr Ile Lys Ala Arg Tyr Glu Ala Leu Lys Asp
   130 135 140
   His Pro Leu Phe Gln Gly Met Thr Tyr Ala Asp Asp Glu Ala Thr Phe
   145 150 155 160
   Thr Glu Lys Leu Pro Leu Met Ala Lys Gly Arg Asp Phe Ser Asp Pro
   165 170 175
   Val Ala Ile Ser Trp Ile Asp Glu Gly Thr Asp Ile Asn Tyr Gly Ala
   180 185 190
   Gln Thr Lys Gln Tyr Leu Asp Ala Ala Glu Val Glu Gly Thr Glu Ile
   195 200 205
   Arg Tyr Gly His Glu Val Lys Ser Ile Lys Ala Asp Gly Ala Lys Trp
   210 215 220
   Ile Val Thr Asp Lys Asn Val His Thr Gly Asp Thr Lys Thr Ile Lys
   225 230 235 240
   Ala Asn Phe Val Phe Val Gly Ala Gly Gly Tyr Ala Leu Asp Leu Leu
   245 250 255
   Arg Ser Ala Gly Ile Pro Gln Val Lys Gly Phe Ala Gly Phe Pro Val
   260 265 270
   Ser Gly Leu Trp Leu Arg Cys Thr Asn Glu Glu Leu Ile Glu Gln His
   275 280 285
   Ala Ala Lys Val Tyr Gly Lys Ala Ser Val Gly Ala Pro Pro Met Ser
   290 295 300
   Val Pro His Leu Asp Thr Arg Val Ile Glu Gly Glu Lys Gly Leu Leu
   305 310 315 320
   Phe Gly Pro Tyr Gly Gly Trp Thr Pro Lys Phe Leu Lys Glu Gly Ser
   325 330 335
   Tyr Leu Asp Leu Phe Lys Ser Ile Arg Pro Asp Asn Ile Pro Ser Tyr
   340 345 350
   Leu Gly Val Ala Ala Gln Glu Phe Asp Leu Thr Lys Tyr Leu Val Thr
   355 360 365
   Glu Val Leu Lys Asp Gln Asp Lys Arg Met Asp Ala Leu Arg Glu Tyr
       370                 375                 380
   Met Pro Glu Ala Gln Asn Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr
                   485                 490
<210> 13
   <211> 20
   <212> DNA
   <213> artificial sequence
<220>
   <223> primer
<220>
   <221> misc_feature
   <222> (1)..(20)
   <223> primer LC-mqo1
<400> 13
   atgaaggcac cgacatcaac 20
<210> 14
   <211> 20
   <212> DNA
   <213> artificial sequence
<220>
   <223> primer
<220>
   <221> misc_feature
   <222> (1)..(20)
   <223> primer LC-mqo2
<400> 14
   tgcaaggcga ttaagttggg 20
<210> 15
   <211> 20
   <212> DNA
   <213> artificial sequence
<220>
   <223> probe
<220>
   <221> misc_feature
   <222> (1)..(20)
   <223> acceptor probe mqo228_C
<220>
   <221> misc_feature
   <222> (10)..(10)
   <223> nucleobase thymine
<400> 15
   acgttcttgt cggtcacgat 20
<210> 16
   <211> 35
   <212> DNA
   <213> artificial sequence
<220>
   <223> probe
<220>
   <221> misc_feature
   <222> (1)..(35)
   <223> donor probe mqo228_A
<400> 16
   gaagtttgcc ttgatggtct tggtgtcgcc agtgt 35
<210> 17
   <211> 23
   <212> DNA
   <213> artificial sequence
<220>
   <223> primer
<220>
   <221> misc_feature
   <222> (1)..(23)
   <223> primer pVW_1.p
<400> 17
   gtgagcggat aacaatttca cac 23
<210> 18
   <211> 20
   <212> DNA
   <213> artificial sequence
<220>
   <223> primer
<220>
   <221> misc_feature
   <222> (1)..(20)
   <223> primer pCV22_2.p
<400> 18
   tgcaaggcga ttaagttggg 20
<210> 19
   <211> 20
   <212> DNA
   <213> artificial sequence
<220>
   <223> primer
<220>
   <221> misc_feature
   <222> (1)..(20)
   <223> primer mqo-E1
<400> 19
   gccttcaact gtgtcagttc 20
<210> 20
   <211> 28
   <212> DNA
   <213> artificial sequence
<220>
   <223> primer
<220>
   <221> misc_feature
   <222> (1)..(28)
   <223> primer mqo-oPl
<400> 20
   gaggatccgc agagaactcg cggagata 28
<210> 21
   <211> 1648
   <212> DNA
   <213> artificial sequence
<220>
   <223> amplificate from Corynebacterium glutamicum strain DM1933_mqo_V228D
<220>
   <221> misc_feature
   <222> (1)..(28)
   <223> nucleotide sequence of primer mqo-oPl
<220>
   <221> misc_feature
   <222> (9)..(1648)
   <223> nucleotide sequence from strain DM1933_mqo_V228D
<220>
   <221> CDS
   <222> (43)..(1542)
<220>
   <221> misc_feature
   <222> (724)..(726)
   <223> gac codon for aspartic acid at position 228
<220>
   <221> misc_feature
   <222> (725)..(725)
   <223> nucleobase adenine
<220>
   <221> misc_feature
   <222> (1270)..(1272)
   <223> ttt codon for phenylalanine at position 410
<220>
   <221> misc_feature
   <222> (1271)..(1271)
   <223> nuclebase thymine
<220>
   <221> misc_feature
   <222> (1543)..(1545)
   <223> stop codon
<220>
   <221> misc_feature
   <222> (1630)..(1648)
   <223> nucleotide sequence of the reverse complement of primer mqo-E1
<400> 21
   gaggatccgc agagaactcg cggagataaa aggaagttga ac atg tca gat tcc          54
                                                  Met Ser Asp Ser
                                                  1
   ccg aag aac gca ccg agg att acc gat gag gca gat gta gtt ctc att        102
   Pro Lys Asn Ala Pro Arg Ile Thr Asp Glu Ala Asp Val Val Leu Ile
   5                   10                  15                  20
   ggt gcc ggt atc atg agc tcc acg ctg ggt gca atg ctg cgt cag ctg        150
   Gly Ala Gly Ile Met Ser Ser Thr Leu Gly Ala Met Leu Arg Gln Leu
                   25                  30                  35
   gag cca agc tgg act cag atc gtc ttc gag cgt ttg gat gga ccg gca        198
   Glu Pro Ser Trp Thr Gln Ile Val Phe Glu Arg Leu Asp Gly Pro Ala
               40                  45                  50
   caa gag tcg tcc tcc ccg tgg aac aat gca gga acc ggc cac tct gct      246
   Gln Glu Ser Ser Ser Pro Trp Asn Asn Ala Gly Thr Gly His Ser Ala
           55                  60                  65
   cta tgc gag ctg aac tac acc cca gag gtt aag ggc aag gtt gaa att      294
   Leu Cys Glu Leu Asn Tyr Thr Pro Glu Val Lys Gly Lys Val Glu Ile
       70                  75                  80
   gcc aag gct gta gga atc aac gag aag ttc cag gtt tcc cgt cag ttc      342
   Ala Lys Ala Val Gly Ile Asn Glu Lys Phe Gln Val Ser Arg Gln Phe
   85                  90                  95                  100
   tgg tct cac ctc gtt gaa gag gga gtg ctg tct gat cct aag gaa ttc      390
   Trp Ser His Leu Val Glu Glu Gly Val Leu Ser Asp Pro Lys Glu Phe
                   105                 110                 115
   atc aac cct gtt cct cac gta tct ttc ggc cag ggc gca gat cag gtt      438
   Ile Asn Pro Val Pro His Val Ser Phe Gly Gln Gly Ala Asp Gln Val
               120                 125                 130
   gca tac atc aag gct cgc tac gaa gct ttg aag gat cac cca ctc ttc      486
   Ala Tyr Ile Lys Ala Arg Tyr Glu Ala Leu Lys Asp His Pro Leu Phe
           135                 140                 145
   cag ggc atg acc tac gct gac gat gaa gct acc ttc acc gag aag ctg      534
   Gln Gly Met Thr Tyr Ala Asp Asp Glu Ala Thr Phe Thr Glu Lys Leu
       150                 155                 160
   cct ttg atg gca aag ggc cgt gac ttc tct gat cca gta gca atc tct      582
   Pro Leu Met Ala Lys Gly Arg Asp Phe Ser Asp Pro Val Ala Ile Ser
   165                 170                 175                 180
   tgg atc gat gaa ggc acc gac atc aac tac ggt gct cag acc aag cag      630
   Trp Ile Asp Glu Gly Thr Asp Ile Asn Tyr Gly Ala Gln Thr Lys Gln
                   185                 190                 195
   tac ctg gat gca gct gaa gtt gaa ggc act gaa atc cgc tat ggc cac      678
   Tyr Leu Asp Ala Ala Glu Val Glu Gly Thr Glu Ile Arg Tyr Gly His
               200                 205                 210
   gaa gtc aag agc atc aag gct gat ggc gca aag tgg atc gtg acc gac      726
   Glu Val Lys Ser Ile Lys Ala Asp Gly Ala Lys Trp Ile Val Thr Asp
           215                 220                 225
   aag aac gta cac act ggc gac acc aag acc atc aag gca aac ttc gtg      774
   Lys Asn Val His Thr Gly Asp Thr Lys Thr Ile Lys Ala Asn Phe Val
       230                 235                 240
   ttc gtc ggc gca ggc gga tac gca ctg gat ctg ctt cgc agc gca ggc      822
   Phe Val Gly Ala Gly Gly Tyr Ala Leu Asp Leu Leu Arg Ser Ala Gly
   245                 250                 255                 260
   atc cca cag gtc aag ggc ttc gct gga ttc cca gta tcc ggc ctg tgg      870
   Ile Pro Gln Val Lys Gly Phe Ala Gly Phe Pro Val Ser Gly Leu Trp
                   265                 270                 275
   ctt cgt tgc acc aac gag gaa ctg atc gag cag cac gca gcc aag gta      918
   Leu Arg Cys Thr Asn Glu Glu Leu Ile Glu Gln His Ala Ala Lys Val
               280                 285                 290
   tat ggc aag gca tct gtt ggc gct cct cca atg tct gtt cct cac ctt      966
   Tyr Gly Lys Ala Ser Val Gly Ala Pro Pro Met Ser Val Pro His Leu
           295                 300                 305
   gac acc cgc gtt atc gag ggt gaa aag ggt ctg ctc ttt gga cct tac       1014
   Asp Thr Arg Val Ile Glu Gly Glu Lys Gly Leu Leu Phe Gly Pro Tyr
       310                 315                 320
   ggt ggc tgg acc cct aag ttc ttg aag gaa ggc tcc tac ctg gac ctg       1062
   Gly Gly Trp Thr Pro Lys Phe Leu Lys Glu Gly Ser Tyr Leu Asp Leu
   325                 330                 335                 340
   ttc aag tcc atc cgc cca gac aac att cct tcc tac ctt ggc gtt gct       1110
   Phe Lys Ser Ile Arg Pro Asp Asn Ile Pro Ser Tyr Leu Gly Val Ala
                   345                 350                 355
   gct cag gaa ttt gat ctg acc aag tac ctt gtc act gaa gtt ctc aag       1158
   Ala Gln Glu Phe Asp Leu Thr Lys Tyr Leu Val Thr Glu Val Leu Lys
               360                 365                 370
   gac cag gac aag cgt atg gat gct ctt cgc gag tac atg cca gag gca       1206
   Asp Gln Asp Lys Arg Met Asp Ala Leu Arg Glu Tyr Met Pro Glu Ala
           375                 380                 385
   caa aac ggc gat tgg gag acc atc gtt gcc gga cag cgt gtt cag gtt       1254
   Gln Asn Gly Asp Trp Glu Thr Ile Val Ala Gly Gln Arg Val Gln Val
       390                 395                 400
   att aag cct gca gga ttt cct aag ttc ggt tcc ctg gaa ttc ggc acc       1302
   Ile Lys Pro Ala Gly Phe Pro Lys Phe Gly Ser Leu Glu Phe Gly Thr
   405                 410                 415                 420
   acc ttg atc aac aac tcc gaa ggc acc atc gcc gga ttg ctc ggt gct       1350
   Thr Leu Ile Asn Asn Ser Glu Gly Thr Ile Ala Gly Leu Leu Gly Ala
                   425                 430                 435
   tcc cct gga gca tcc atc gca cct tcc gca atg atc gag ctg ctt gag       1398
   Ser Pro Gly Ala Ser Ile Ala Pro Ser Ala Met Ile Glu Leu Leu Glu
               440                 445                 450
   cgt tgc ttc ggt gac cgc atg atc gag tgg ggc gac aag ctg aag gac       1446
   Arg Cys Phe Gly Asp Arg Met Ile Glu Trp Gly Asp Lys Leu Lys Asp
           455                 460                 465
   atg atc cct tcc tac ggc aag aag ctt gct tcc gag cca gca ctg ttt       1494
   Met Ile Pro Ser Tyr Gly Lys Lys Leu Ala Ser Glu Pro Ala Leu Phe
       470                 475                 480
   gag cag cag tgg gca cgc acc cag aag acc ctg aag ctt gag gaa gcc       1542
   Glu Gln Gln Trp Ala Arg Thr Gln Lys Thr Leu Lys Leu Glu Glu Ala
   485                 490                 495                 500
   taaatcttct aactgctttc tttaaagcac ccgcacatgt ctgttgaggt ttcacctgcg     1602
   gagacaatct ccgccttcat gggttggaac tgacacagtt gaaggc                    1648
<210> 22
   <211> 500
   <212> PRT
   <213> artificial sequence
<220>
   <223> Synthetic Construct
<400> 22 201700371A 2

## Description

L-lysine is used in human medicine, in the pharmaceutical industry, in the food industry and particularly in nutrition of animals.

It is known that L-lysine is produced by fermentation of strains of the species *Corynebacterium glutamicum.* Because of the great economic importance, work is continually being done on improving the production methods. Improvements may relate to the fermentation technology such as e. g. stirring and supplying oxygen, or to the composition of the nutrient media e.g. the sugar concentration during fermentation, or to the processing of the fermentation broth to a suitable product form by e. g. drying and granulating the fermentation broth or ion exchange chromatography or may relate to the intrinsic performance properties of the microorganism itself.

The methods used for improving the performance properties of these microorganisms are those of mutagenesis, selection and screening of mutants. The strains obtained in this way are resistant to anti-metabolites or are auxotrophic for metabolites of regulatory importance, and produce L-lysine. A well-known anti-metabolite is the L-lysine analogue S-(2-aminoethyl)-L-cysteine (see e.g. Tosaka et al.: Agricultural and Biological Chemistry 42(4), 745-752, (1978)).

Methods of recombinant DNA technology have likewise been used for a number of years for improvement of L-lysine-producing strains of the species *Corynebacterium glutamicum,* by modifying, i.e. enhancing or attenuating, individual L-lysine biosynthesis genes and investigating the effect on L-lysine production.

The nucleotide sequences of the chromosomes of various bacteria or strains resp. of the species *Corynebacterium glutamicum,* and their analysis have been disclosed. This information is available at publicly accessible data bases and may be used for strain development purposes. One such data base is the GenBank data base of the NCBI (National Center for Biotechnology Information, U.S. National Library of Medicine 8600 Rockville Pike, Bethesda MD, 20894 USA).

During the annotation procedure for a sequenced chromosome of an organism identified structures such as e. g. genes or coding sequences are furnished with a unique identifier called locus_tag by the supplier of the information to the data base.

The nucleotide sequence of the *Corynebacterium glutamicum* ATCC13032 chromosome and its analysis were described by Ikeda and Nakagawa (Applied Microbiology and Biotechnology 62, 99-109(2003)) and in EP1108790A2. The information is available at the NCBI under accession number NC_003450. In the chromosome sequence disclosed under accession number NC_003450 locus_tag NCgl1926 identifies a nucleotide sequence coding for a malate:quinone oxidoreductase. The amino acid sequence of the polypeptide is available under the identifier NP_60120.

The nucleotide sequence of the *Corynebacterium glutamicum* ATCC13032 chromosome and its analysis were independently described by Kalinowski et al. (Journal of Biotechnology 104 (1-3), 5-25 (2003)). The information is available at the NCBI under accession number NC_006958. Locus_tag CGTRNA_RS09800 identifies a nucleotide sequence coding for a malate:quinone oxidoreductase. The old_locus_tag designation cg2192 is also used in the art. The amino acid sequence of the polypeptide is available under the identifier WP_011014814.

The nucleotide sequences of locus_tag NCgl1926 and CGTRNA_RS09800 are identical.

Lv et al. (Journal of Bacteriology 194(3), 742-743 (2012) describe the sequencing of the chromosome of *Corynebacterium glutamicum* ATCC14067, a strain formerly referred to as *Brevibacterium flavum.* The information is available at the NCBI under accession number AGQQ02000001 and AGQQ02000002. The nucleotide sequence of the chromosome of *Corynebacterium glutamicum* ATCC13869, a strain formerly referred to as *Brevibacterium lactofermentum,* and its analysis were disclosed by Chen et al. at the NCBI under accession number NZ_CP016335.

Malate:quinone oxidoreductase is a membrane associated enzyme catalyzing the oxidation of malate to oxaloacetate with quinones serving as electron acceptors. The biochemical and genetic characterization of the enzyme of *Corynebacterium glutamicum* and its function were described by Molenaar et al. (European Journal of 254, 395-403 (1998)) and Molenaar et al. (Journal of Bacteriology 182(24), 6884-6891 (2000)). Molenaar et al. referred to the enzyme as EC 1.1.99.16. According to current IUBMB Enzyme Nomenclature the enzyme is given the systematic designation EC 1.1.5.4 with malate dehydrogenase (quinone) as accepted name and (*S*)-malate:quinone oxidoreductase as systematic name. The art uses the abbreviation Mqo for the enzyme or for the polypeptide resp.. The gene encoding the Mqo polypeptide is called *mqo.* Information concerning transcription signals, e.g. -10 region of a promoter, the 5'-untranslated leader sequence (5'-UTR), and translation signals, e.g. ribosome binding site (RBS), of the *mqo* gene (old_locus_tag cg2192) can be found in Pfeifer-Sancar et al. (BMC Genomics 14:888 (2013)) and Han et al. (Journal of Molecular Microbiology and Biotechnology 15:264-276, 2008).

US20020028490A1 describes the favorable effect of overexpression of malate:quinone oxidoreductase on L-lysine and L-threonine production by *Corynebacterium glutamicum.*

WO02086137A2 describes the favorable effect of attenuating or eliminating the malate:quinone oxidoreductase activity on L-lysine production by *Corynebacterium glutamicum.*

WO03029457A1 likewise describes the favorable effect of attenuating or eliminating the malate:quinone oxidoreductase activity on L-lysine production by *Corynebacterium glutamicum.*

US20060166338A1 describes the favorable effect of an amino acid exchange at position 111 of the amino acid sequence of the malate:quinone oxidoreductase on L-lysine production in *Corynebacterium glutamicum.*

Object of the present invention is to provide new measures for the fermentative production of L-lysine by bacteria of the species *Corynebacterium glutamicum.*

To achieve the object outlined above the present invention makes available novel L-lysine excreting bacteria of the species *Corynebacterium glutamicum,* having the ability to excrete L-lysine, containing in their chromosome a polynucleotide encoding a polypeptide having the activity of a malate:quinone reductase wherein the amino acid at position 228 of the amino acid sequence of the polypeptide contains any proteinogenic amino acid different from valine.

The present invention further makes available methods for producing said L-lysine by using said bacteria in a fermentative process and for the manufacturing of a product containing said L-lysine from the fermentation broth.

The objects underlying the present invention were solved by the means as written in the claims.

Accordingly the present invention provides the following:
A bacterium of the species *Corynebacterium glutamicum,* having the ability to excrete L-lysine, containing in its chromosome a polynucleotide encoding a polypeptide having the activity of a malate:quinone oxidoreductase and comprising the amino acid sequence of SEQ ID NO:2, wherein the amino acid valine at position 228 is substituted by a different proteinogenic amino acid, preferably by aspartic acid or glutamic acid, particular preferred by aspartic acid.

It was found that the bacteria modified according to the invention excreted L-lysine, into a suitable medium under suitable fermentation conditions in an increased manner with respect to one or more parameters selected from product concentration (i.e. amount of L-lysine produced per volume, or mass unit resp., of medium/fermentation broth (e.g. g/l or g/kg)), product yield (i.e. amount of L-lysine produced per carbon source consumed (e.g. g/g or kg/kg)), product formation rate (i.e. amount of L-lysine produced per volume, or mass unit resp., of medium/fermentation broth and unit of time (e.g. g/l x h or g/kg x h)), and specific product formation rate (i.e. amount of L-lysine produced per unit of time and mass unit of the producer (e.g. g/h x g dry mass)) as compared to the unmodified bacterium.

It is obvious that a higher product concentration facilitates product manufacturing e. g. purification and isolation. An increased product yield reduces the amount of raw material required. An increased product formation rate reduces the time required for a fermentation run thus increasing the availability of a given fermenter.

The bacteria according to the invention thus contribute to the improvement of technical and economic aspects of the manufacturing of L-lysine or L-lysine containing products.

In a further embodiment the bacterium according to the invention contains in its chromosome a polynucleotide encoding an amino acid sequence of a polypeptide having malate:quinone oxidoreductase activity comprising the amino acid sequence according to SEQ ID NO:2, wherein the amino acid valine at position 228 of the encoded amino acid sequence of SEQ ID NO:2 is substituted by aspartic acid, and comprising the nucleotide sequence of positions 1001 to 2500 of SEQ ID NO:1 the nucleobase at position 1683 being adenine or comprising the nucleotide sequence of positions 1001 to 2500 of SEQ ID NO:3.

In a further embodiment the bacterium according to the invention contains in its chromosome a polynucleotide which is coding for an amino acid sequence of a polypeptide having malate:quinone oxidoreductase activity comprising the amino acid sequence according to SEQ ID NO:2, wherein the amino acid valine at position 228 of the encoded amino acid sequence of SEQ ID NO:2 is substituted by a different proteinogenic amino acid, preferably by aspartic acid or glutamic acid, particularly preferred by aspartic acid, and wherein the 5'-end of the coding sequence of this polynucleotide is directly connected to the 3'-end of the polynucleotide comprising the nucleotide sequence from positions 781 to 1000 of SEQ ID NO:1 or of SEQ ID NO:3. In particular, the bacterium of the species *Corynebacterium glutamicum* according to the present invention contains in its chromosome a polynucleotide encoding the polypeptide comprising the amino acid sequence according to SEQ ID NO:2 the amino acid at position 228 being aspartic acid and having the activity of a malate:quinone oxidoreductase which comprises the nucleotide sequence from positions 781 to 2500 of SEQ ID NO:3 or the nucleotide sequence from positions 781 to 2500 of SEQ ID NO:1 the nucleobase at position 1683 being adenine. In other words, the bacterium according to the present invention may contain in its chromosome a polynucleotide encoding said amino acid sequence which comprises the nucleotide sequence from positions 781 to 2500 of SEQ ID NO:1 whereby the nucleobase at position 1683 is adenine or the nucleotide sequence from positions 781 to 2500 of SEQ ID NO:3.

The term L-lysine, where mentioned herein, in particular in the context of product formation, also comprises their ionic forms and salts, for example L-lysine mono hydrochloride or L-lysine sulfate.

For practicing the present invention bacteria of the species *Corynebacterium glutamicum* are used. A description of the genus *Corynebacterium* and the species *Corynebacterium glutamicum* comprised by this genus can be found in the article *"Corynebacterium"* by K. A. Bernard and G. Funke in Bergey's Manual of Systematics of Archaea and Bacteria (Bergey's Manual Trust, 2012).

Suitable strains of *Corynebacterium glutamicum* are wild strains of this species for example strains ATCC13032, ATCC14067 and ATCC13869, and L-lysine excreting strains obtained from these wild strains.

Strain ATCC13032 (also available as DSM20300) is the taxonomic type strain of the species *Corynebacterium glutamicum.* Strain ATCC14067 (also available as DSM20411) is also known under the outdated designation *Brevibacterium flavum.* Strain ATCC13869 (also available as DSM1412) is also known under the outdated designation *Brevibacterium lactofermentum.* A taxonomic study of this group of bacteria based on DNA-DNA hybridization was done by Liebl et al. (International Journal of Systematic Bacteriology 41(2), 255-260, 1991). A comparative analysis of various strains of the species *Corynebacterium glutamicum* based on genome sequence analysis was provided by Yang and Yang (BMC Genomics 18(1):940).

A multitude of L-lysine excreting strains of the genus *Corynebacterium,* in particular of the species *Corynebacterium glutamicum* were obtained in the art during the past decades starting from strains such as ATCC13032, ATCC14067, ATCC13869 and the like. They were obtained as a result of strain development programs using inter alia methods like classical mutagenesis, selection for antimetabolite resistance as well as amplification and promotor modification of genes of the biosynthetic pathway of the L-amino acid in question by genetic engineering methods. Summaries may be found in L. Eggeling and M. Bott (Handbook of Corynebacterium glutamicum, CRC Press, 2005) or H. Yukawa and M. Inui (Corynebacterium glutamicum Biology and Biotechnology, Springer Verlag, 2013).

L-lysine excreting strains of the species *Corynebacterium glutamicum* are widely known in the art and can be used for the purpose of the present invention. For example Blombach et al. (Applied and Environmental Microbiology 75(2), 419-427, 2009) describe strain DM1933, which is deposited under accession DSM25442 according to the Budapest treaty. Strain DM1933 was obtained from ATCC13032 by several steps of strain development. Furthermore L-lysine excreting *Corynebacterium glutamicum* strain DM2031, deposited according to the Budapest Treaty as DSM32514 may be used. Strain DM2031 is a further developed derivative of DM1933 having enhanced L-lysine excretion ability. Other L-lysine excreting *Corynebacterium glutamicum* strains are e. g. described in WO2008033001A1 and EP0841395A1.

L-lysine excreting strains of the species *Corynebacterium glutamicum* typically contain a polynucleotide coding for a feedback resistant aspartokinase polypeptide variant. A feedback resistant aspartokinase polypeptide variant means an aspartokinase which is less sensitive, or desensitized resp., to inhibition by mixtures of L-lysine and L-threonine, e.g. 10 mM each, or mixtures of the L-lysine analogue S-(2-aminoethyl)-L-cysteine and L-threonine, e.g. 50 mM S-(2-aminoethyl)-L-cysteine and 10 mM L-threonine, when compared to the wild form of the enzyme, which is contained in wild strains like for example ATCC13032, ATCC14067 and ATCC13869. The EC number for aspartokinase is EC 2.7.2.4. Descriptions of polynucleotides of *Corynebacterium glutamicum* encoding a feedback resistant aspartokinase polypeptide variant are for example given in US5688671, US6844176 and US6893848. A summarizing list can be found inter alia in US20090311758A1. The symbol used in the art for a gene coding for an aspartokinase polypeptide is *lysC.* In case the gene codes for a feedback resistant polypeptide variant the art typically uses symbols like *lysC^{fbr}* with fbr indicating feedback resistance.

Accordingly said L-lysine excreting strains of the species *Corynebacterium glutamicum* used for the measures of the present invention preferably contain at least one copy of a polynucleotide coding for a feedback resistant aspartokinase polypeptide.

SEQ ID NO:5 shows the nucleotide sequence of the coding sequence of the aspartokinase polypeptide of strain ATCC13032 and SEQ ID NO:6 the amino acid sequence of the encoded polypeptide. It is known in the art (see US6893848) that exchange of the amino acid Thr at position 311 of SEQ ID NO:6 for Ile imparts the enzyme feedback resistance to inhibition by mixtures of L-lysine and L-threonine.

Accordingly it is preferred that the amino acid sequence of said feedback resistant aspartokinase polypeptide comprises the amino acid sequence of SEQ ID NO:6 containing isoleucine at position 311.

Said amino exchange can be achieved by exchanging the nucleobase cytosine (c) at position 932 of SEQ ID NO:5 to give thymine (t). The acc codon for threonine is thus altered to the atc codon for isoleucine.

It is further known in the art that exchange of the gtg start codon of the coding sequence for the aspartokinase polypeptide for atg enhances expression of the polypeptide (see e.g. EP2796555A2).

Accordingly it is preferred that the sequence coding for a feedback resistant aspartokinase polypeptide begins with an atg start codon.

Summaries concerning the breeding of L-lysine excreting strains of *Corynebacterium glutamicum* may be found inter alia in L. Eggeling and M. Bott (Handbook of Corynebacterium glutamicum, CRC Press, 2005), V. F. Wendisch (Amino Acid Biosynthesis - Pathways, Regulation and Metabolic Engineering, Springer Verlag, 2007), H. Yukawa and M. Inui (Corynebacterium glutamicum Biology and Biotechnolgy, Springer Verlag, 2013) or A. Yokota and M. Ikeda (Amino Acid Fermentation, Springer Verlag, 2017), and Eggeling and Bott (Applied Microbiology and Biotechnology 99 (9), 3387-3394, 2015).

In case a wild strain, e.g. ATCC13032, ATCC13869 or ATCC14067, is in a first step subjected to the measures of the present invention the resulting strain is in a second step subjected to a strain development program targeted at L-lysine in order to obtain a bacterium according to the present invention.

The term DSM denotes the depository Deutsche Sammlung für Mikroorganismen und Zellkulturen located in Braunschweig, Germany. The term ATCC denotes the depository American Type Culture Collection located in Manasass, Virginia, US.

Details regarding the biochemistry and chemical structure of polynucleotides and polypeptides as present in living things such as bacteria like *Corynebacterium glutamicum* or *Escherichia coli,* for example, can be found inter alia in the text book "Biochemie" by Berg et al. (Spektrum Akademischer Verlag Heidelberg, Berlin, Germany, 2003; ISBN 3-8274-1303-6).

Polynucleotides consisting of deoxyribonucleotide monomers containing the nucleobases or bases resp. adenine (a), guanine (g), cytosine (c) and thymine (t) are referred to as deoxyribopolynucleotides or deoxyribonucleic acid (DNA). Polynucleotides consisting of ribonucleotide monomers containing the nucleobases or bases resp. adenine (a), guanine (g), cytosine (c) and uracil (u) are referred to as ribo-polynucleotides or ribonucleic acid (RNA). The monomers in said polynucleotides are covalently linked to one another by a 3',5'-phosphodiester bond. By convention single strand polynucleotides are written from 5'- to 3'-direction. Accordingly a polynucleotide has a 5'-end and 3'-end. The order of the nucleotide monomers in the polynucleotide is commonly referred to as nucleotide sequence. Accordingly a polynucleotide is characterized by its nucleotide sequence. For the purpose of this invention deoxyribopolynucleotides are preferred. In bacteria, for example *Corynebacterium glutamicum* or *Escherichia coli,* the DNA is typically present in double stranded form. Accordingly the length of a DNA molecule is typically given in base pairs (bp). The nucleotide sequence coding for a specific polypeptide is called coding sequence (cds). A triplett of nucleotides specifying an amino acid or a stop signal for translation is called a codon. The codons specifying different amino acids are well known in the art.

A gene from a chemical point of view is a polynucleotide, preferably a deoxyribopolynucleotide.

DNA can be synthesized *de novo* by the phoshoramidite method (McBride and Caruthers: Tetrahedon Letters 24(3) 245- 248 (1983)). Summaries concerning *de novo* DNA synthesis may be found in the review article of Kosuri and Chruch (Nature Methods 11(5), 499 - 507 (2014)) or in the article of Graf et al. in the book "Systems Biology and Synthetic Biology" by P. Fu and S. Panke (John Wiley, US, 2009, pp 411-438).

The term gene refers to a polynucleotide comprising a nucleotide sequence coding for a specific polypeptide (coding sequence) and the adjoining stop codon. In a broader sense the term includes regulatory sequences preceding and following the coding sequence. The preceding sequence is located at the 5'-end of the coding sequence and is also referred to as upstream sequence. A promotor is an example of a regulatory sequence located 5' to the coding sequence. The sequence following the coding sequence is located at its 3'-end and also referred to as downstream sequence. In the context of the present invention said regulatory sequence, comprising inter alia the promotor and the ribosome binding site, of the mqo gene preceding its coding sequence is located from position 781 to 1000 of SEQ ID NO:1 or SEQ ID NO:3. A transcriptional terminator is an example of a regulatory sequence located 3' to the coding sequence.

Polypeptides consist of L-amino acid monomers joined by peptide bonds. For abbreviation of L-amino acids the one letter code and three letter code of IUPAC (International Union of Pure and Applied Chemistry) is used. Due to the nature of polypeptide biosynthesis polypeptides have an amino terminal end and a carboxyl terminal end also referred to as N-terminal end and C-terminal end. The order of the L-amino acids or L-amino acid residues resp. in the polypeptide is commonly referred to as amino acid sequence. Polypeptides are also referred to as proteins.
Further it is known in the art that the start codon or initiation codon resp. gtg of a coding sequence as well as atg encodes the amino acid methionine. It is known in the art that the N-terminal amino acid methionine of an encoded polypeptide may be removed by an aminopeptidase during or after translation (Jocelyn E. Krebs, Elliott S. Goldstein and Stephan T. Kilpatrick: Lewin's Genes X, Jones and Bartlett Publishers, US, 2011).

Proteinogenic L-amino acids are to be understood to mean the L-amino acids present in natural proteins, that is proteins of microorganisms, plants, animals and humans. Proteinogenic L-amino acids comprise L-aspartic acid, L-asparagine, L-threonine, L-serine, L-glutamic acid, L-glutamine, L-glycine, L-alanine, L-cysteine, L-valine, L-methionine, L-isoleucine, L-leucine, L-tyrosine, L-phenylalanine, L-histidine, L-lysine, L-tryptophan, L-arginine, L-proline and in some cases L-selenocysteine and L-pyrrolysine. It is common in the ass to refer to these proteinogenic L-amino acids without hinting to the L configuration at the α carbon atom of the L-amino acid; e.g. L-valine may simply called valine, L-aspartic acid may simply be called aspartic acid etc..

Teachings and information concerning the handling of and experimental work with polynucleotides may be found inter alia in the handbook of J. Sambrook et al. (Molecular Cloning, A Laboratory Manual, Cold Spring Harbor Laboratory Press, 1989), the textbook of C. R. Newton and A. Graham (PCR, Spektrum Akademischer Verlag, 1994) and the handbook of D. Rickwood and B. D. Hames (Gel electrophoresis of nucleic acids, a practical approach, IRL Press, 1982).

For sequence analysis of polynucleotides and polypeptides, e.g. sequence alignments the Clustal W program (Larkin et al.: Clustal W and Clustal X version 2.0. In: Bioinformatics 23, 2947-2948 (2007)) or public software such as the CLC Genomics Workbench (Qiagen, Hilden, Germany) or the program MUSCLE provided by the European Bioinformatics Institute (EMBL-EBI, Hinxton, UK) may be used.

*Corynebacterium glutamicum,* in particular strain ATCC13032 and L-lysine excreting strains obtained therefrom during a strain development program, contain in their chromosome among others a gene encoding a polypeptide having the activity of a malate:quinone oxidoreductase and comprising the amino acid sequence of SEQ ID NO:2. The coding sequence is shown SEQ ID NO:1 positions 1001 to 2500. The coding sequence may contain silent mutations which do not alter the amino acid sequence of the Mqo polypeptide. For example the amino acid Phe at position 410 of the amino acid sequence shown in SEQ ID NO:2 or SEQ ID NO:4 may be specified by the codon ttc or by the codon ttt (see positions 2228 to 2230, in particular position 2230, of SEQ ID NO:1 or SEQ ID NO:3). This context is also known as degeneracy of the genetic code in the art.

During the work for the present invention it was found that modifying L-lysine excreting bacteria of the species *Corynebacterium glutamicum* by exchanging the amino acid valine at position 228 of the encoded amino acid sequence of the Mqo polypeptide shown in SEQ ID NO:2 for a different proteinogenic, preferably aspartic acid or glutamic acid, particular preferred aspartic acid, increased their ability to excrete L-lysine as compared to the unmodified bacterium.

The skilled artisan is aware of a number of methods of mutagenesis how to achieve said modification in the *Corynebacterium glutamicum.*

A mutant bacterium according to the invention can be obtained by classical *in vivo* mutagenesis executed with cell populations of strains of *Corynebacterium glutamicum* using mutagenic substances, e.g. N-methyl-N'-nitro-N-nitrosoguanidine, or ultra violet light. The nucleotide sequence comprising the site of mutagenesis within the *mqo* gene can be amplified by PCR using primers selected from SEQ ID NO:1 or SEQ ID NO:3. By sequencing the PCR product the desired mutants are identified. Details concerning this approach can be found inter alia in US7754446.

A further method is the CRISPR-Cpf1 assisted genome editing described by Jiang et al. (Nature Communications 8: 15179 DOI: 10; 1038/ncomms15179).

Another common method of mutating genes of *Corynebacterium glutamicum* is the method of gene replacement described by Schwarzer and Pühler (Bio/Technology 9, 84-87 (1991)) and further elaborated by Schafer et al. (Gene 145, 69-73 (1994)).

Peters-Wendisch et al. (Microbiology 144, 915-927 (1998)) used the gene replacement method to inactivate the *pyc* gene of *Corynebacterium glutamicum* encoding pyruvate carboxylase. Schafer et al. used the method to incorporate a deletion into the *hom-thrB* gene region of *Corynebacterium glutamicum.* In EP1094111 the method was used to incorporate a deletion into the *pck* gene of *Corynebacterium glutamicum* encoding phosphoenol pyruvate carboxykinase. In US7585650 the method was applied to the *zwf* gene to realize an amino acid exchange at position 321 of the amino acid sequence of the Zwf sub-unit of the glucose 6-phosphate dehydrogenase. In US7754446 the method was applied to the *rel* gene to realize an amino acid exchange at position 38 of the amino acid sequence of the GTP- pyrophosphate kinase polypeptide.

In the gene replacement method, a mutation, for example, a deletion, insertion or substitution of at least one nucleobase, is provided by an isolated polynucleotide comprising the nucleotide sequence of the gene in question or a part thereof containing the mutation.

In the context of the present invention the nucleotide sequence of the gene in question is the *mqo* gene.

In the context of the present invention the mutation is a substitution of at least one nucleobase located in the codon specifying the amino acid valine at position 228 of the encoded amino acid sequence (see SEQ ID NO:1 and SEQ ID NO:3) of the Mqo polypeptide.

As a consequence of said mutation the codon specifies a proteinogenic amino acid different from valine, preferably aspartic acid or glutamic acid, particular preferred aspartic acid. The codons specifying aspartic acid are gat or gac. The codon gac is preferred.

The codon for the amino acid at position 228 has the position from 1682 to 1684 in SEQ ID NO:1 or SEQ ID NO:3. The nucleotide sequence from position 1682 to 1684, in particular the nucleotide at position 1683, may also be referred to as site of mutation.

The mutated nucleotide sequence of the gene in question or a part thereof containing the mutation comprises i) a nucleotide sequence at the 5'-end of the site of mutation, which is also referred to as 5'-flanking sequence or upstream sequence in the art, ii) a nucleotide sequence at the 3'-end of the site of mutation, which is also referred to as 3'-flanking sequence or downstream sequence in the art, and iii) the nucleotide sequence of the site of mutation between i) and ii).

Said 5'-flanking sequence and 3'-flanking sequence required for homologous recombination typically have a length of at least 200 bp, at least 400 bp, at least 600 bp or at least 800 bp. The maximum length typically is 1000 bp, 1500 bp or 2000 bp.

An example of a mutated nucleotide sequence in the context of the present invention comprises the nucleotide sequence shown in SEQ ID NO:1 from positions 1083 to 2283 with adenine at position 1683. Another example of a mutated nucleotide sequence in the context of the present invention comprises the nucleotide sequence shown in SEQ ID NO:3 from positions 1083 to 2283.

A further example of a mutated nucleotide sequence in the context of the present invention comprises the nucleotide sequence shown in SEQ ID NO:7. The nucleotide sequence of SEQ ID NO:7 corresponds to SEQ ID NO:3 from positions 882 to 2484. SEQ ID NO:7 contains a part of the coding sequence of the mutated *mqo* gene. Said part of the coding sequence present in SEQ ID NO:7 encodes the N'-terminal end of the Mqo polypeptide i.e. the amino acids from positions 1 to 494. Said part of the coding sequence lacks the sequence coding for the c-terminal end of the Mqo polypeptide (see SEQ ID NO:8).

The 5'-flanking sequence comprises the nucleotide sequence from positions 1 to 801 of SEQ ID NO:7. The 3'-flanking sequence consists of the nucleotide sequence from positions 803 to 1603 of SEQ ID NO:7. The site of mutation is at position 802.

The mutated nucleotide sequence provided is cloned into a plasmid vector that is not capable of autonomous replication in *Corynebacterium glutamicum.* Said plasmid vector comprising said mutated nucleotide sequence is subsequently transferred into the desired strain of *Corynebacterium glutamicum* by transformation or conjugation. After two events of homologous recombination comprising a recombination event within the 5'-flanking sequence provided by the plasmid vector with the homologous sequence of the *Corynebacterium glutamicum* chromosome and a recombination event within the 3'-flanking sequence provided by the plasmid vector with the homologous sequence of the *Corynebacterium glutamicum* chromosome, one effecting integration and one effecting excision of said plasmid vector, the mutation is incorporated in the *Corynebacterium glutamicum* chromosome. Thus the nucleotide sequence of the gene in question contained in the chromosome of said desired strain is replaced by the mutated nucleotide sequence.

An event of homologous recombination may also be referred to as crossing over.

It is preferred that the L-lysine excreting *Corynebacterium glutamicum* strains of the present invention have the ability to excrete ≥ 0,25 g/l, preferably ≥ 0,5 g/l, particularly preferred ≥ 1,0 g/l of L-lysine in a suitable medium under suitable conditions.

The invention further provides a fermentative process for producing L-lysine, using the *Corynebacterium glutamicum* of the present invention.

In a fermentative process according to the invention a *Corynebacterium glutamicum* modified in accordance with the present invention and having the ability to excrete L-lysine is cultivated in a suitable medium under suitable conditions. Due to said ability to excrete said L-lysine the concentration of the L-lysine increases and accumulates in the medium during the fermentative process and the L-lysine is thus produced.

The fermentative process may be discontinuous process like a batch process or a fed batch process or a continuous process. A summary concerning the general nature of fermentation processes is available in the textbook by H. Chmiel (Bioprozesstechnik, Spektrum Akademischer Verlag, 2011), in the textbook of C. Ratledge and B. Kristiansen (Basic Biotechnology, Cambridge University Press,2006) or in the textbook of V.C. Hass and R. Pörtner (Praxis der Bioprozesstechnik Spektrum Akademischer Verlag, 2011).

A suitable medium used for the production of L-lysine by a fermentative process contains a carbon source, a nitrogen source, a phosphorus source, inorganic ions and other organic compounds as required.

Suitable carbon sources include glucose, fructose, sucrose as well as the corresponding raw materials like starch hydrolysate, molasses or high fructose corn syrup.

As nitrogen source organic nitrogen-containing compounds such as peptones, meat extract, soy bean hydrolysates or urea, or inorganic compounds such as ammonium sulphate, ammonium chloride, ammonium phosphate, ammonium carbonate, ammonium nitrate, ammonium gas or aqueous ammonia can be used.
As phosphorus source, phosphoric acid, potassium dihydrogen phosphate or dipotassium hydrogen phosphate or the corresponding sodium-containing salts can be used.

Inorganic ions like potassium, sodium, magnesium, calcium, iron and further trace elements etc. are supplied as salts of sulfuric acid, phosphoric acid or hydrochloric acid.

Other organic compounds means essential growth factors like vitamins e. g. thiamine or biotin or L-amino acids e. g. L-homoserine.

The media components may be added to the culture in form of a single batch or be fed in during the cultivation in a suitable manner.

During the fermentative process the pH of the culture can be controlled by employing basic compounds such as sodium hydroxide, potassium hydroxide, ammonia or aqueous ammonia, or acidic compounds such as phosphoric acid or sulphuric acid in a suitable manner. The pH is generally adjusted to a value of from 6.0 to 8.5, preferably 6.5 to 8.0. To control foaming, it is possible to employ antifoam agents such as, for example, fatty acid polyglycol esters. To maintain the stability of plasmids, it is possible to add to the medium suitable selective substances such as, for example, antibiotics. The fermentative process is preferably carried out under aerobic conditions. In order to maintain these conditions, oxygen or oxygen-containing gas mixtures such as, for example air are introduced into the culture. The fermentative process is carried out, where appropriate, at elevated pressure, for example at an elevated pressure of 0.03 to 0.2 MPa. The temperature of the culture is normally from 25 °C to 40 °C, preferably from 30 °C to 37 °C. In a discontinuous process, the cultivation is continued until an amount of the L-lysine sufficient for being recovered has been formed. The cultivation is then completed. This aim is normally achieved within 10 hours to 160 hours. In continuous processes, longer cultivation times are possible. Examples of suitable media and culture conditions can be found inter alia in L. Eggeling and M. Bott (Handbook of Corynebacterium glutamicum, CRC Press, 2005) and the patent documents US5770409, US5990350, US 5275940, US5763230 and US6025169.

Thus the fermentative process results in a fermentation broth which contains the desired L-lysine.

Accordingly a method for the fermentative production of L-lysine is provided comprising the steps of
a) cultivating a *Corynebacterium glutamicum* of the present invention in a suitable medium under suitable conditions, and
b) accumulating said L-lysine in the medium to produce an L-lysine containing fermentation broth.

A product containing the L-lysine is then recovered in liquid or solid from the fermentation broth.

A "fermentation broth" means a medium in which a *Corynebacterium glutamicum* of the invention has been cultivated for a certain time and under certain conditions.

When the fermentative process is completed, the resulting fermentation broth accordingly comprises:
a) the biomass (cell mass) of the *Corynebacterium glutamicum* of the invention, said biomass having been produced due to propagation of the cells of said *Corynebacterium glutamicum,*
b) the desired L-lysine accumulated during the fermentative process,
c) the organic by-products accumulated during the fermentative process, and
d) the components of the medium employed which have not been consumed in the fermentative process.

The organic by-products include compounds which may be formed by the *Corynebacterium glutamicum* of the invention during the fermentative process in addition to production of the L-lysine.

The fermentation broth is removed from the culture vessel or fermentation tank, collected where appropriate, and used for providing a product containing the L-lysine, in liquid or solid form. The expression "recovering the L-lysine-containing product" is also used for this. In the simplest case, the L-lysine -containing fermentation broth itself, which has been removed from the fermentation tank, constitutes the recovered product.

The fermentation broth can subsequently be subjected to one or more of the measures selected from the group consisting of:
a) partial (>0% to <80%) to complete (100%) or virtually complete (≥ 80%, ≥ 90%, ≥ 95%, ≥ 96%, ≥ 97%, ≥ 98%, ≥ 99%) removal of the water,
b) partial (>0% to <80%) to complete (100%) or virtually complete (≥ 80%, ≥ 90%, ≥ 95%, ≥ 96%, ≥ 97%, ≥ 98%, ≥ 99%) removal of the biomass, the latter being optionally inactivated before removal,
c) partial (>0% to <80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the organic by-products formed during the fermentative process, and
d) partial (>0% to <80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the residual components of the medium employed or of the residual input materials resp., which have not been consumed in the fermentative process.

An abundance of technical instructions for measures a), b), c) and d) are available in the art.

Removal of water (measure a)) can be achieved inter alia by evaporation, using e.g. a falling film evaporator, by reverse osmosis or nanofiltration. The concentrates thus obtained can be further worked up by spray drying or spray granulation. It is likewise possible to dry the fermentation broth directly using spray drying or spray granulation.

Removal of the biomass (measure b)) can be achieved inter alia by centrifugation, filtration or decantation or a combination thereof.

Removal of the organic by-products (measure c)) or removal of residual components of the medium (measure d) can be achieved inter alia by chromatography, e.g. ion exchange chromatography, treatment with activated carbon or crystallization. In case the organic by-products or residual components of the medium are present in the fermentation broth as solids they can be removed by measure b).

General instructions on separation, purification and granulation methods can be found inter alia in the book of R. Ghosh "Principles of Bioseperation Engineering" (World Scientific Publishing, 2006), the book of F. J. Dechow "Seperation and Purification Techniques in Biotechnology" (Noyes Publications, 1989), the article "Bioseparation" of Shaeiwitz et al. (Ullmann's Encyclopedia of Industrial Chemistry, Wiley-VCH, 2012) and the book of P. Serno et al. "Granulieren" (Editio Cantor Verlag, 2007).

A downstream processing scheme for L-lysine products can be found in the article "L-lysine Production" of R. Kelle et al. (L. Eggeling and M. Bott (Handbook of Corynebacterium glutamicum, CRC Press, 2005)). US5279744 teaches the manufacturing of a purified L-lysine product by ion exchange chromatography. US5431933 teaches the manufacturing of dry L-amino acid products, e. g. an L-lysine product, containing most of the constituents of the fermentation broth.

Thus a concentration or purification of the L-lysine is achieved and a product having the desired content of said L-lysine is provided.

Finally the the L-lysine may be isolated from the culture broth and crystallized in form of a salt, preferably in form of the hydrochloric acid salt of L-lysine.

Analysis of L-lysine to determine its concentration at one or more time(s) during the fermentation can take place by separating the L-lysine by means of ion exchange chromatography, preferably cation exchange chromatography, with subsequent post-column derivatization using ninhydrin, as described in Spackman et al. (Analytical Chemistry 30: 1190-1206 (1958)). It is also possible to employ ortho-phthalaldehyde rather than ninhydrin for post-column derivatization. An overview article on ion exchange chromatography can be found in Pickering (LC.GC (Magazine of Chromatographic Science 7(6):484-487 (1989)). It is likewise possible to carry out a pre-column derivatization, for example using ortho-phthalaldehyde or phenyl isothiocyanate, and to fractionate the resulting amino acid derivates by reversed-phase chromatography (RP), preferably in the form of high-performance liquid chromatography (HPLC). A method of this type is described, for example, in Lindroth et al. (Analytical Chemistry 51:1167-1174 (1979)). Detection is carried out photometrically (absorption, fluorescence). A review regarding amino acid analysis can be found inter alia in the textbook "Bioanalytik" by Lottspeich and Zorbas (Spektrum Akademischer Verlag, Heidelberg, Germany 1998).

### EXPERIMENTAL SECTION

### A) MATERIALS and METHODS

The molecular biology kits, primers and chemicals used and some details of the methods applied are briefly described herewith.

### 1. Antibiotics and chemicals

a. Kanamycin: Kanamycin solution from *Streptomyces kanamyceticus* from Sigma Aldrich (St. Louis, USA, Cat. no. K0254).
b. Nalidixic acid: Nalidixic acid sodium salt from Sigma Aldrich (St. Louis, USA, Cat. no. N4382).
c. If not stated otherwise, all chemicals were purchased analytically pure from Merck (Darmstadt, Germany), Sigma Aldrich (St. Louis, USA) or Carl-Roth (Karlsruhe, Germany).

### 2. Cultivation

If not stated otherwise, all cultivation / incubation procedures were performed as follows:
a. LB broth (MILLER) from Merck (Darmstadt, Germany; Cat. no. 110285) was used to cultivate *E. coli* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 37°C and 200 rpm.
b. LB agar (MILLER) from Merck (Darmstadt, Germany Cat. no. 110283) was used for cultivation of *E. coli* strains on agar plates. The agar plates were incubated at 37°C in an INCU-Line® mini incubator from VWR (Radnor, USA).
c. Brain heart infusion broth (BHI) from Merck (Darmstadt, Germany; Cat. no. 110493) was used to cultivate *C*. *glutamicum* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 33°C and 200 rpm.
d. Brain heart agar (BHI-agar) from Merck (Darmstadt, Germany; Cat. no. 113825) was used for cultivation of *C. glutamicum* strains on agar plates. The agar plates were incubated at 33°C in an incubator from Heraeus Instruments with Kelvitron® temperature controller (Hanau, Germany).

### 3. Determining optical density

a. The optical density of bacterial suspensions in shake flask cultures was determined at 600 nm (OD600) using the BioPhotometer from Eppendorf AG (Hamburg, Germany).
b. The optical density of bacterial suspensions produced in the Wouter Duetz (WDS) micro fermentation system (24-Well Plates) was determined at 660 nm (OD660) with the GENios™ plate reader from Tecan Group AG (Männedorf, Switzerland).

### 4. Centrifugation

a. Benchtop centrifuge for reaction tubes with a volume up to 2 ml
   Bacterial suspensions with a maximum volume of 2 ml were caused to sediment using 1 ml or 2 ml reaction tubes (e.g. Eppendorf Tubes® 3810X) using an Eppendorf 5417 R centrifuge (5 min. at 13.000 rpm).
b. Benchtop centrifuge for tubes with a volume up to 50 ml
   Bacterial suspensions with a maximum volume of 50 ml were caused to sediment using 15 ml or 50 ml centrifuge tubes (e.g. Falcon™ 50 ml Conical Centrifuge Tubes) using an Eppendorf 5810 R centrifuge for 10 min. at 4.000 rpm.

### 5. DNA isolation

a. Plasmid DNA was isolated from E. coli cells using the QIAprep Spin Miniprep Kit from Qiagen (Hilden, Germany, Cat. No. 27106).
b. Total DNA from *C. glutamicum* was isolated using the method of Eikmanns et al. (Microbiology 140, 1817-1828, 1994).

### 6. Polymerase chain reaction (PCR)

PCR with a proof reading (high fidelity) polymerase was used to amplify a desired segment of DNA before assembly or Sanger sequencing.
a. The Phusion® High-Fidelity DNA Polymerase Kit (NEB Phusion Kit) from New England BioLabs Inc. (Ipswich, USA; Cat. No. M0530) was used for template-correct amplification of selected DNA regions according to the instructions of the manufacturer (see table 1).

**Table 1: Thermocycling conditions for PCR with Phusion® High-Fidelity DNA Polymerase Kit from NEB Inc.**

| PCR-program | | | |
|---|---|---|---|
| Step | Time [min.] | T [°C] | Description |
| 1 | 00:30 | 98 | Initial denaturation step |
| 2 | 00:05 | 98 | Denaturation step |
| 3 | 00:30 | 60 | Annealing step |
| 4 | 00:xx | 72 | Elongation step 15-30 sec. per kb DNA |
| | | | Repeat step 2 to 4: 30 x |
| 5 | 05:00 | 72 | Final Elongation step |
| 6 | Hold | 4 | Cooling step |

b. Primer
The oligonucleotides used were synthesized by eurofins genomics GmbH (Ebersberg, Germany) using the phosphoramidite method described by McBride and Caruthers (Tetrahedron Lett. 24, 245-248, 1983).
c. Template
As PCR template either a suitably diluted solution of isolated plasmid DNA or of isolated total DNA from a *C. glutamicum* liquid culture or the total DNA contained in a colony was used (colony PCR). For said colony PCR the template was prepared by taking cell material with a toothpick from a colony on an agar plate and placing the cell material directly into the PCR reaction tube. The cell material was heated for 10 sec. with 800 W in a microwave oven type Mikrowave & Grill from SEVERIN Elektrogeräte GmbH (Sundern, Germany) and then the PCR reagents were added to the template in the PCR reaction tube.
d. PCR Cycler
PCR's were carried out in PCR Cyclers type Mastercycler or Mastercycler nexus gradient from Eppendorf AG (Hamburg, Germany).

### 7. Detection of mutations using FRET

The presence of a given mutation, e.g. a nucleobase exchange, was detected by real-time PCR in combination with FRET hybridization probes. The term FRET is the abbreviation for fluorescence resonance energy transfer. As real-time PCR instrument a Lightcycler from Roche Diagnostics® was used (see below).

This method was e. g. used by M. J. Lay and C. T. Wittwer (Clinical Chemistry 42 (12), 2262-2267 (1997)) for the genotyping of factor V Leiden. Cyril DS Mamotte (The Clinical Biochemist Reviews 27, 63-75 (2006) reviews the genotyping of single nucleotide substitutions using this method. Summaries concerning this method may be found in the textbooks Lewin's Genes XII by Jocelyn E. Krebs, Elliott S. Goldstein and Stephan T. Kilpatrick (Jones and Bartlett Publishers, US, 2018), Molecular Diagnostics, 12 Tests that changed everything by W. Edward Highsmith (Humana Press, Springer, New York, 2014) or elsewhere in the art.
The FRET hybridization donor probe was labelled with the fluorescent dye fluorescein and the acceptor probe with the fluorescent dye LC-Red640. In essence the detection method comprised three steps: colony PCR, probe hybridization and subsequent melting curve analysis. The method is simply referred to as real-time PCR herewith.
a. Primers and Probes
The oligonucleotides used were synthesized by eurofins genomics GmbH (Ebersberg, Germany).
b. Template
As PCR template the total DNA contained in a colony was used. It was prepared by taking cell material with a toothpick from a colony on an agar plate and placing the cell material directly into the PCR reaction tube. The cell material was heated for 10 sec. with 800 W in a microwave oven type Mikrowave & Grill from SEVERIN Elektrogeräte GmbH (Sundern, Germany) and then the PCR reagents were added to the template in the PCR reaction tube.
e. Reaction Mix
The Type-it® Fast SNP probe PCR Kit (Type-it Kit) from Qiagen (Hilden, Germany, Cat.No. 206045) was used for real-time detection of the mutations. Therefore 2.5 µl of the Qiagen Fast SNP Puffer (2x) was mixed with 0.5 µl of each of the LC-PCR-Primers [10 µM] and 0.5 µl of each of the 1:500 diluted acceptor and donor probe [100 pmol/µl] to get the mastermix for the real-time PCR.

**Table 2: Thermocycling conditions for PCR with the LightCycler® (step 1-3) and melting curve analysis (step 4-6).**

| PCR-program | | | |
|---|---|---|---|
| Step | Time [sec.] | T [°C] | Description |
| 1 | 15 | 95 | Denaturation step (and Activation of HotStarTaq™ DNA polymerase) |
| 2 | 05 | 55 | Annealing step |
| 3 | 30 | 72 | Elongation step |
| | | | Repeat step 1 to 3: 50 x |
| 4 | 10 | 95 | Denaturation step |
| 5 | 30 | 40 | Probe hybridisation |
| 6 | | 40- 80 | Melting curve analysis |
| 7 | | 80- 40 | Cooling |

f. PCR Cycler
The reactions were carried out in a LightCycler® 2.0 Instrument and analysed with LightCycler® Software 4.1 of Roche Diagnostics (Rotkreuz, Switzerland).

### 8. Restriction enzyme digestion of DNA

The FastDigest restriction endonucleases (FD) and the associated buffer from ThermoFisher Scientific (Waltham, USA) were used for restriction digestion of plasmid DNA. The reactions were carried out according to the instructions of the manufacturer's manual.

### 9. Determining the size of DNA fragments

The size of DNA fragments was determined by automatic capillary electrophoresis using the QIAxcel from Qiagen (Hilden, Germany).

### 10. Purification of PCR amplificates and restriction fragments

PCR amplificates and restriction fragments were cleaned up using the QIAquick PCR Purification Kit from Qiagen (Hilden, Germany; Cat. No. 28106), according to the manufacturer's instructions.

### 11. Determining DNA concentration

DNA concentration was measured using the NanoDrop Spectrophotometer ND-1000 from PEQLAB Biotechnologie GmbH, since 2015 VWR brand (Erlangen, Germany).

### 12. Gibson Assembly

Vectors allowing integration of the desired mutation into the chromosome were made using the method of Gibson et al. (Science 319, 1215-20, 2008). The Gibson Assembly Kit from New England BioLabs Inc. (Ipswich, USA; Cat. No. E2611) was used for this purpose. The reaction mix, containing the restricted vector and at least one DNA insert, was incubated at 50°C for 60 min.. 0.5 µl of the Assembly mixture was used for a transformation experiment.

### 13. Chemical transformation of E. coli

a. Chemically competent *E. coli* Stellar™ cells were purchased from Clontech Laboratories Inc. (Mountain View, USA; Cat. No. 636763) and transformed according to the manufacturer's protocol (PT5055-2).
   These cells were used as transformation hosts for reaction mixtures obtained by Gibson Assembly. The transformation batches were cultivated overnight for approximately 18 h at 37°C and the transformants containing plasmids selected on LB agar supplemented with 50 mg/l kanamycin.
b. *E. coli* K-12 strain S17-1 was used as donor for conju-gational transfer of plasmids based on pK18mobsacB from *E. coli* to *C*. *glutamicum.* Strain S17-1 is described by Simon, R. et al. (Bio/Technology 1, 784-794, 1983). It is available from the American Type Culture Collection under the access number ATCC47055.

Chemically competent *E. coli* S17-1 cells were made as follows: A preculture of 10 ml LB medium (10 ml liquid medium per 100 ml Erlenmeyer flask with 3 baffles) was inoculated with 100 µl bacterial suspension of strain S17-1 and the culture was incubated overnight for about 18 h at 37°C and 250 rpm. The main culture (70 ml LB contained in a 250 ml Erlenmeyer flask with 3 baffles) was inoculated with 300 µl of the preculture and incubated up to an OD600 of 0.5-0.8 at 37°C. The culture was centrifuged for 6 min. at 4°C and 4000 rpm and the supernatant was discarded. The cell pellet was resuspended in 20 ml sterile, ice-cold 50 mM CaCl₂ solution and incubated on ice for 30 min.. After another centrifugation step, the pellet was resuspended in 5 ml ice-cold 50 mM CaCl₂ solution and the suspension incubated on ice for 30 min.. The cell suspension was then adjusted to a final concentration of 20 % glycerol (v/v) with 85 % (v/v) sterile ice-cold glycerol. The suspension was divided into 50 µl aliquots and stored at -80°C.

To transform S17-1 cells, the protocol according to Tang et al. (Nucleic Acids Res. 22(14), 2857-2858, 1994) with a heat shock of 45 sec. was used.

### 14. Conjugation of C. glutamicum

The pK18mobsacB plasmid system described by Schafer et al. (Gene 145, 69 - 73, 1994) was used to integrate desired DNA fragments into the chromosome of *C. glutamicum.* A modified conjugation method of Schafer et al. (Journal of Bacteriology 172, 1663 - 1666, 1990) was used to transfer the respective plasmid into the desired *C. glutamicum* recipient strain.

Liquid cultures of the *C. glutamicum* strains were carried out in BHI medium at 33°C. The heat shock was carried out at 48.5°C for 9 min.. Transconjugants were selected by plating the conjugation batch on EM8 agar (Table 4), which was supplemented with 25 mg/l kanamycin and 50 mg/l nalidixic acid. The EM8 agar plates were incubated for 72 h at 33°C.

**Table 4: Composition of the EM8 agar**

| Components | Concentration (g/l) |
|---|---|
| Glucose (sterile-filtered) | 23 |
| CSL (corn steep liquor) | 30 |
| Peptone from soymeal (Merck, Germany) | 40 |
| (NH₄)₂SO₄ | 8 |
| Urea | 3 |
| KH₂PO₄ | 4 |
| MgSO₄ · 7 H₂O | 0.5 |
| FeSO₄ · 7 H₂O | 0.01 |
| CuSO₄ · 5 H₂O | 0.001 |
| ZnSO₄ · 7 H₂O | 0.01 |
| Calcium pantothenate, D(+) | 0.01 |
| Thiamine | 0.001 |
| Inositol | 0.1 |
| Nicotinic acid | 0.001 |
| Biotin (sterile-filtered) | 0.005 |
| CaCO₃ (autoclaved separately) | 1.6 |
| Agar-Agar (Merck, Germany) | 14 |

Sterile toothpicks were used to transfer the transconjugants onto BHI agar, which was supplemented with 25 mg/l kanamycin and 50 mg/l nalidixic acid. The agar plates were incubated for 20 h at 33°C. The cultures of the respective transconjugants produced in this manner were then propagated further for 24 h at 33°C in 10 ml BHI medium contained in 100 ml Erlenmeyer flasks with 3 baffles. An aliquot was taken from the liquid culture suitably diluted and plated (typically 100 to 200 µl) on BHI agar which was supplemented with 10% saccharose. The agar plates were incubated for 48 h at 33°C. The colonies growing on the saccharose containing agar plates were then examined for the phenotype kanamycin sensitivity. To do so a toothpick was used to remove cell material from the colony and to transfer it onto BHI agar containing 25 mg/l kanamycin and onto BHI agar containing 10% saccharose. The agar plates were incubated for 60 h at 33°C. Clones that proved to be sensitive to kanamycin and resistant to saccharose were examined for integration of the desired DNA fragment by means of real-time PCR.

### 15. Determining nucleotide sequences

Nucleotide sequences of DNA molecules were determined by eurofins genomics GmbH (Ebersberg, Germany) by cycle sequencing, using the dideoxy chain termination method of Sanger et al. (Proceedings of the National Academy of Sciences USA 74, 5463 - 5467, 1977), on Applied Biosystems® (Carlsbad, CA, USA) 3730xl DNA Analyzers. Clonemanager Professional 9 software from Scientific & Educational Software (Denver, USA) was used to visualise and evaluate the sequences.

### 16. Glycerol stocks of E.coli and C. glutamicum strains

For long time storage of *E.coli-* and *C*. *glutamicum* strains glycerol stocks were prepared. Selected *E. coli* clones were cultivated in 10 ml LB medium supplemented with 2 g/l glucose. Selected *C. glutamicum* clones were cultivated in two fold concentrated BHI medium supplemented with 2 g/l glucose. Cultures of plasmid containing *E*. *coli* strains were supplemented with 50 mg/l kanamycin. Cultures of plasmid containing *C. glutamicum* strains were supplemented with 25 mg/l kanamycin. The medium was contained in 100 ml Erlenmeyer flasks with 3 baffles. It was inoculated with a loop of cells taken from a colony and the culture incubated for about 18 h at 37°C and 200 rpm in the case of *E*. *coli* and 33°C and 200 rpm in the case of *C. glutamicum.* After said incubation period 1.2 ml 85% (v/v) sterile glycerol were added to the culture. The obtained glycerol containing cell suspension was then aliquoted in 2 ml portions and stored at -80°C.

### 17. Cultivation system according to Wouter Duetz (WDS)

The millilitre-scale cultivation system according to Duetz (Trends Microbiol. 2007; 15(10):469-75) was used to investigate the performance of the *C. glutamicum* strains constructed. For this purpose, 24-deepwell microplates (24 well WDS plates) from EnzyScreen BV (Heemstede, Netherlands; Cat. no. CR1424), filled with 2.5 mL medium were used.
Precultures of the strains were done in 10 ml two fold concentrated BHI medium. The medium was contained in a 100 ml Erlenmeyer flask with 3 baffles. It was inoculated with 100 µl of a glycerol stock culture and the culture incubated for 24 h at 33°C and 200 rpm.
After said incubation period the optical densities OD600 of the precultures were determined. The main cultures were done by inoculating the 2.5 ml medium containing wells of the 24 Well WDS-Plate with an aliquot of the preculture to give an optical density OD600 of 0.1.

As medium for the main culture CGXII medium described by Keilhauer et al. (J. Bacteriol. 1993 Sep; 175(17): 5595-5603) was used. For convenience the composition of the CGXII medium is shown in table 8.

**Table 8: Composition of Keilhauer's CGXII medium.**

| Components | Concentration (g/l) |
|---|---|
| MOPS (3-(N-Morpholino)propanesulfonic acid) | 42 |
| (NH₄)₂SO₄ | 20 |
| Urea | 5 |
| KH₂PO₄ | 1 |
| K₂HPO₄ | 1 |
| MgSO₄ · 7 H₂O | 0.25 |
| CaCl₂ | 0.01 |
| FeSO₄ · 7 H₂O | 0.01 |
| MnSO₄ H₂O | 0.01 |
| ZnSO₄ · 7 H₂O | 0.001 |
| CuSO₄ · 5 H₂O | 0.0002 |
| NiCl₂ 6 H₂O | 0.00002 |
| Biotin (sterile-filtered) | 0.0002 |
| Protocatechuic acid (sterile-filtered) | 0.03 |
| Carbon source (sterile-filtered) | as needed |
| adjust the pH to 7 with NaOH | |

These main cultures were incubated for approximately 45 h at 33 °C and 300 rpm in an Infors HT Multitron standard incubator shaker from Infors GmbH (Bottmingen, Switzerland) until complete consumption of glucose.
The glucose concentration in the suspension was analysed with the blood glucose-meter OneTouch Vita® from LifeScan (Johnson & Johnson Medical GmbH, Neuss, Germany).

After cultivation the culture suspensions were transferred to a deep well microplate. A part of the culture suspension was suitably diluted to measure the OD600. Another part of the culture was centrifuged and the concentration of L-amino acids, in particular L-lysine, and residual glucose were analysed in the supernatant.

### 18. Amino acid analyser

The concentration of L-lysine and other L-amino acids, e.g. L-valine, in the culture supernatants was determined by ion exchange chromatography using a SYKAM S433 amino acid analyser from SYKAM Vertriebs GmbH (Fürstenfeldbruck, Germany). As solid phase a column with spherical, polystyrene-based cation exchanger (Peek LCA N04/Na, dimension 150 x 4.6 mm) from SYKAM was used. Depending on the L-amino acid the separation takes place in an isocratic run using a mixture of buffers A and B for elution or by gradient elution using said buffers. As buffer A an aquous solution containing in 20 l 263 g trisodium citrate, 120 g citric acid, 1100 ml methanol, 100 ml 37 % HCI and 2 ml octanoic acid (final pH 3.5) was used. As buffer B an aquous solution containing in 20 I 392 g trisodium citrate, 100 g boric acid and 2 ml octanoic acid (final pH 10.2) was used. The free amino acids were coloured with ninhydrin through post-column derivatization and detected photometrically at 570 nm.

### 19. Glucose determination with continuous flow system (CFS)

A SANplus multi-channel continuous flow analyser from SKALAR analytic GmbH (Erkelenz, Germany) was used to determine the concentration of glucose in the supernatant. Glucose was detected with a coupled-enzyme assay (Hexokinase/ Glucose-6-Phosphate-Dehydrogenase) via NADH formation.

### B) EXPERIMENTAL RESULTS

### Example 1

### Sequence of the mqo gene of C. glutamicum strain DM1933 and DM2463

Strain DM1933 is an L-lysine producer described by Blombach et al. (Applied and Environmental Microbiology 75(2), 419-427, 2009). It is deposited according to the Budapest treaty at the DSMZ under accession number DSM25442.
Strain DM2463 is an L-lysine producer obtained from ATCC13032 during a multi step strain breeding program aiming at increased performance parameters for L-lysine production.
The nucleotide sequence of the chromosome of strain DM1933 and of strain DM2463 was determined by Illumina whole-genome sequencing technology (Illumina Inc., San Diego, CA, US). See e.g. Benjak et al. (2015) Whole-Genome Sequencing for Comparative Genomics and De Novo Genome Assembly. In: Parish T., Roberts D. (eds) Mycobacteria Protocols. Methods in Molecular Biology, Vol 1285. Humana Press, NY, US) and Bennet, S. (Pharmacogenomics 5(4), 433-438, 2004).

It was found that the nucleotide sequence of the *mqo* coding sequence of strain DM1933 including the nucleotide sequence upstream and downstream thereof is identical to that of ATCC13032 shown in SEQ ID NO:1.
It was found that the nucleotide sequence of the *mqo* coding sequence of strain DM2463 including the sequence of 1000 nucleobases upstream and 1003 nucleobases downstream thereof is different in two positions to that of ATCC13032 shown in SEQ ID NO:1. Instead of nucleobase thymine at position 1683 of SEQ ID NO:1 the corresponding nucleobase in strain DM2463 is adenine. Instead of nucleobase cytosine at position 2230 of SEQ ID NO:1 the corresponding nucleobase in strain DM2463 is thymine.
As a consequence of the mutation at position 1683 the amino acid at position 228 of the encoded amino acid sequence of the Mqo polypeptide of strain DM2463 is aspartic acid (abbreviated as D or Asp). The amino acid at position 228 of the encoded amino acid sequence of the Mqo polypeptide of strain ATCC13032 is valine (abbreviated as V or Val) as shown in SEQ ID NO:2. This alteration or exchange resp. in the amino acid sequence from valine to aspartic acid is abbreviated as V228D.
The mutation at position 2230 is a silent mutation. It does not lead to an alteration of the amino acid at position 410 of the encoded amino acid sequence, which is phenylalanine.
The nucleotide sequence of the *mqo* coding sequence of strain DM2463 including the nucleotide sequence upstream and downstream thereof is also shown in SEQ ID NO:3. The amino acid sequence of the encoded Mqo polypeptide of strain DM2463 is shown in SEQ ID NO:4.
DM1933 contains in its chromosome a variant of the aspartokinase gene encoding a feed back resistant aspartokinase polypeptide. Said feed back resistant aspartokinase polypeptide has the amino acid sequence of SEQ ID NO:6 of the sequence listing, wherein the amino acid threonine (Thr) at position 311 of the amino acid sequence is replaced by isoleucine (IIe). In US 7,338,790 the abbreviation "lysC T3111" is used to indicate said exchange. Blombach et al. use the abbreviation "lysC(T311l)".

### Example 2

### Construction of plasmid pK18mobsacB_mqo'_V228D

Plasmid pK18mobsacB_mqo'_V228D was constructed to enable incorporation of the mutation causing the amino acid exchange V228D (see example 1) into the nucleotide sequence of the *mqo* coding sequence of strain DM1933. The plasmid is based on the mobilizable vector pK18mobsacB described by Schafer et al. (Gene 145, 69-73, 1994). For the construction of pK18mobsacB_mqo'_V228D the Gibson Assembly method was used.
For this purpose two polynucleotides or DNA molecules resp. were made: One polynucleotide called mqo'_V228D comprised the 5'-end of the *mqo* coding sequence including the mutation leading to the amino acid exchange of valine at position 228 to aspartate (V228D). The second polynucleotide was plasmid pK18mobsacB linearized by treatment with restriction endonuclease *Xbal.*

Polynucleotide mqo'_V228D was synthesized by PCR using total DNA isolated from a C. *glutamicum* DM2463 culture as template and oligonucleotides 1f-mqo-pK18 and 1r-mqo-pK18 as primers (table 9). The primers are also shown in SEQ ID NO:9 and SEQ ID NO:10 of the sequence listing. For PCR the Phusion Kit was used with an elongation step (see table 4, step 4) of 30 sec.

**Table 9: List of primers used and size of amplificate during Phusion Kit PCR.**

| synthesis of amplificate | name | sequence | size [bp] |
|---|---|---|---|
| mqo'_V228D | 1f-mqo-pK18 | | 1634 |
| | 1r-mqo-pK18 | | |

The nucleotide sequence of the amplificate mqo'_V228D is shown in SEQ ID NO:11. It contains the nucleotide sequence shown in SEQ ID NO:7.
Amplificate mqo'_V228D contains a sequence of 801 bps length upstream and a sequence of 801 bps downstream from the mutation (see position 818 of SEQ ID NO:11 or position 802 of SEQ ID NO:7) causing the amino acid exchange from valine (V) to aspartic acid (D) in strain DM2463.
At its 5'-end and 3'-end it is equipped with a nucleotide sequence each overlapping with the corresponding sequence of pK18mobsacB cut with *XbaI.*
Said overlapping sequences are required for the Gibson assembly technique.
Plasmid pK18mobsacB was linearized with the restriction endonuclease *XbaI.* The digestion mixture was subsequently controlled by capillary electrophoresis, purified and the DNA concentration quantified.
To assemble the plasmid pK18mobsacB_mqo'_V228D the two polynucleotides i.e. the vector pK18mobsacB cut with *XbaI* and the amplificate mqo'_V228D were mixed with the Gibson Assembly Kit ingredients. The assembly mixture thus obtained was used to transform chemically competent *E. coli* Stellar™ cells.
22 kanamycin resistant transformants were analyzed by real-time PCR (see table 2) using the Type-it Kit and the primers LC-mqo1 and LC-mqo2 for PCR amplification and oligonucleotides mqo228_C as acceptor probe and mqo228_A as donor probe for melting curve analysis. The primers and probes are shown in table 10 and under SEQ ID NO:13 to SEQ ID NO:16 of the sequence listing.

**Table 10: List of primers and probes used for real-time PCR.**

| name | sequence |
|---|---|
| LC-mqo1 | ATGAAGGCACCGACATCAAC |
| LC-mqo2 | TGCAAGGCGATTAAGTTGGG |
| mqo228_C¹ | ACGTTCTTGTCGGTCACGAT |
| mqo228_A² | GAAGTTTGCCTTGATGGTCTTGGTGTCGCCAGTGT |

| | |
|---|---|
| ¹ acceptor probe labelled with LC-Red640 at the 5'-end and phosphorylated at the 3'-end ² donor probe labelled with fluorescein at the 3'-end | |

Plasmid DNA from three transformants thus characterized as containing the desired mutation was isolated and the polynucleotide mqo'_V228D created within the plasmid during the Gibson assembly analyzed by Sanger sequencing. For sequence analysis the primers pVW_1.p, pCV22_2.p (table 11), LC-mqo1 and LC-mqo2 (table 10) were used. They are also shown under SEQ ID NO:17 and 18 and SEQ ID NO:13 and 14 of the sequence listing.

**Table 11: List of primers used for Sanger sequencing.**

| analysis of | name | sequence |
|---|---|---|
| mqo'_V228D | pVW_1.p | GTGAGCGGATAACAATTTCACAC |
| | pCV22_2.p | TGCAAGGCGATTAAGTTGGG |

The analysis of the nucleotide sequences thus obtained showed that the polynucleotide mqo'_V228D contained in the plasmid of the three transformants analyzed had the nucleotide sequence presented in SEQ ID NO:11. One of said transformants was called Stellar/ pK18mobsacB_mqo'_V228D and saved as a glycerol stock for further experiments.

### Example 3

### Construction of strain DM1933_mqo_V228D

The plasmid pK18mobsacB_mqo'_V228D obtained in example 2 was used to incorporate the mutation (see position 818 of SEQ ID NO:11) leading to the amino acid exchange V228D (see SEQ ID NO:12) into the chromosome of the L-lysine producer DM1933.
Chemically competent cells of *E*. *coli* strain S17-1 were transformed with plasmid DNA of pK18mobsacB_mqo'_V228D. The modified conjugation method of Schafer et al. (Journal of Bacteriology 172, 1663 - 1666, 1990) as described in materials and methods was used for conjugal transfer into the strain DM1933 and for selection of transconjugant clones by virtue of their saccharose resistance and kanamycin sensitivity phenotype.
Transconjugant clones were analyzed by real-time PCR using the Type-it Kit and the primers LC-mqo1 and LC-mqo2 for PCR amplification and mqo228_C as acceptor probe and mqo228_A as donor probe for melting curve analysis (table 10).
One of the transconjugant clones thus characterized was called DM1933_mqo_V228D. A glycerol stock culture of the transconjugant clone was prepared and used as starting material for further investigations.
The nucleotide sequence of the chromosomal region of strain DM1933_mqo_V228D containing the mutated nucleotide sequence was analyzed by Sanger sequencing.
For this purpose a PCR amplificate was produced spanning the site of mutation. A colony PCR was done using the primers mqo-E1 and mqo-oP1 (see table 12) and the NEB Phusion Kit with an elongation time of 45 sec. (table 1, step 4). The amplificate obtained was then sequenced using the primers LC-mqo1 and LC-mqo2 (see table 10). The nucleotide sequences of the primers used in this context are also shown in SEQ ID NO:19 and 20.

**Table 12: List of primers used for colony PCR.**

| amplification/detection of | name | sequence | size [bp] |
|---|---|---|---|
| mqo_V228D | mqo-E1 | GCCTTCAACTGTGTCAGTTC | 1648 |
| | mqo-oP1 | GAGGATCCGCAGAGAACTCGCGGAGATA | |

The nucleotide sequence obtained is shown in SEQ ID NO:21. The result showed that strain DM1933_mqo_V228D contained the desired mutation, or the desired mutated nucleotide sequence resp., in its chromosome.
Thus the *mqo* gene of strain DM1933 was mutated with the effect that the amino acid valine at position 228 of the amino acid sequence of the encoded Mqo polypeptide was replaced by aspartic acid.

### Example 4

### L-lysine production by strain DM1933_mqo_V228D

Strains DM1933 (reference) and DM1933_mqo_V228D obtained in example 3 were analyzed for their ability to produce L-lysine from glucose by batch cultivation using the cultivation system according to Wouter Duetz.
As medium CGXII containing 20 g/l glucose as carbon source was used. The cultures were incubated for 45 h until complete consumption of glucose as confirmed by glucose analysis using blood glucose-meter and the concentrations of L-lysine and the optical density OD660 were determined. The result of the experiment is presented in table 14.

**Table 14: L-lysine production by strain DM1933_mqo_V228D.**

| strain | L-lysine¹ (g/l) | OD660 |
|---|---|---|
| DM1933 | 3.9 | 8.3 |
| DM1933_mqo_V228D | 4.3 | 8.0 |

| | | |
|---|---|---|
| ¹ as L-lysine x HCl | | |

The experiment shows that L-lysine production was increased in strain DM1933_mqo_V228D as compared to the parent strain DM1933.

## Claims

1. A bacterium of the species *Corynebacterium glutamicum* having the ability to excrete L-lysine containing in its chromosome a polynucleotide encoding a polypeptide having the activity of a malate:quinone oxidoreductase and comprising the amino acid sequence of SEQ ID NO:2, wherein the amino acid valine at position 228 is substituted by a different proteinogenic amino acid.

2. The bacterium as claimed in claim 1, wherein said amino acid at position 228 of the amino acid sequence of SEQ ID NO:2 is aspartic acid or glutamic acid.

3. The bacterium as claimed in claims 1 or 2, wherein said amino acid at position 228 of the amino acid sequence of SEQ ID NO:2 is aspartic acid.

4. The bacterium as claimed in claim 3, wherein the polynucleotide encoding said amino acid sequence comprises the nucleotide sequence of positions 1001 to 2500 of SEQ ID NO:1 the nucleobase at position 1683 being adenine.

5. The bacterium as claimed in claim 3, wherein the polynucleotide encoding said amino acid sequence comprises the nucleotide sequence of positions 1001 to 2500 of SEQ ID NO:3.

6. The bacterium of any of the claims 1 to 5, wherein the 5'-end of the polynucleotide encoding said amino acid sequence is directly connected to the 3'-end of the polynucleotide comprising the nucleotide sequence from positions 781 to 1000 of SEQ ID NO:1 or of SEQ ID NO:3.

7. The bacterium as claimed in claim 6, wherein the polynucleotide encoding said amino acid sequence comprises the nucleotide sequence from positions 781 to 2500 of SEQ ID NO:3.

8. The bacterium as claimed in claim 6, wherein the polynucleotide encoding said amino acid sequence comprises the nucleotide sequence from positions 781 to 2500 of SEQ ID NO:1 the nucleobase at position 1683 being adenine.

9. A method for the fermentative production of an L-lysine comprising the steps of
a) cultivating the bacterium as defined in any of claims 1 to 8 in a
suitable medium under suitable conditions, and
b) accumulating L-lysine in the medium to
form an L-lysine containing fermentation broth.

10. The method as claimed in claim 9, wherein said method comprises manufacturing an L-lysine containing product from said fermentation broth.

11. The method as claimed in any of the claims 9 to 10, wherein said method comprises extracting or substantially eliminating water from said fermentation broth.

12. The method of claim 11, wherein at least 40 % (w/w), preferred at least 90 % (w/w), more preferred at least 95 % (w/w) water are extracted from the fermentation broth.

13. The method of claim 12, wherein said manufacturing comprises a purification step.

14. The method of claim 13, wherein said purification step is selected from the group consisting of treatment with char coal, ionic exchange and crystallization.

15. The method of claim 10, further comprising isolation of L-lysine from the fermentation broth and crystallization of L-lysine in form of a salt.

## Patentansprüche

1. Bakterium der Art *Corynebacterium glutamicum,* das die Fähigkeit hat, L-Lysin auszuscheiden, und das in seinem Chromosom ein Polynukleotid enthält, das ein Polypeptid mit der Aktivität einer Malat:Chinon-Oxidoreduktase codiert und die Aminosäuresequenz von SEQ ID NO: 2 umfasst, wobei die Aminosäure Valin an Position 228 durch eine andere proteinogene Aminosäure substituiert ist.

2. Bakterium nach Anspruch 1, wobei die Aminosäure an Position 228 der Aminosäuresequenz von SEQ ID NO: 2 Asparaginsäure oder Glutaminsäure ist.

3. Bakterium nach den Ansprüchen 1 oder 2, wobei die Aminosäure an Position 228 der Aminosäuresequenz von SEQ ID NO: 2 Asparaginsäure ist.

4. Bakterium nach Anspruch 3, wobei das die Aminosäuresequenz codierende Polynukleotid die Nukleotidsequenz der Positionen 1001 bis 2500 von SEQ ID NO: 1 umfasst, wobei die Nukleobase an Position 1683 Adenin ist.

5. Bakterium nach Anspruch 3, wobei das die Aminosäuresequenz codierende Polynukleotid die Nukleotidsequenz der Positionen 1001 bis 2500 von SEQ ID NO: 3 umfasst.

6. Bakterium nach einem der Ansprüche 1 bis 5, wobei das 5'-Ende des die Aminosäuresequenz codierenden Polynukleotids direkt mit dem 3'-Ende des Polynukleotids verbunden ist, das die Nukleotidsequenz von den Positionen 781 bis 1000 von SEQ ID NO : 1 oder von SEQ ID NO: 3 umfasst.

7. Bakterium nach Anspruch 6, wobei das die Aminosäuresequenz codierende Polynukleotid die Nukleotidsequenz von den Positionen 781 bis 2500 von SEQ ID NO: 3 umfasst.

8. Bakterium nach Anspruch 6, wobei das die Aminosäuresequenz codierende Polynukleotid die Nukleotidsequenz von den Positionen 781 bis 2500 von SEQ ID NO: 1 umfasst, wobei die Nukleobase an Position 1683 Adenin ist.

9. Verfahren zur fermentativen Produktion eines L-Lysins, umfassend die Schritte
a) Kultivieren des Bakteriums nach einem der Ansprüche 1 bis 8 in einem geeigneten Medium unter geeigneten Bedingungen und
b) Anreichern von L-Lysin in dem Medium zur Bildung einer L-Lysin-haltigen Fermentationsbrühe.

10. Verfahren nach Anspruch 9, wobei das Verfahren die Herstellung eines L-Lysin-haltigen Produkts aus der Fermentationsbrühe umfasst.

11. Verfahren nach einem der Ansprüche 9 bis 10, wobei das Verfahren das Extrahieren oder das wesentliche Entfernen von Wasser aus der Fermentationsbrühe umfasst.

12. Verfahren nach Anspruch 11, wobei mindestens 40% (Gew./Gew.), vorzugsweise mindestens 90% (Gew./Gew.), stärker bevorzugt mindestens 95% (Gew./Gew.) Wasser aus der Fermentationsbrühe extrahiert werden.

13. Verfahren nach Anspruch 12, wobei die Herstellung einen Reinigungsschritt umfasst.

14. Verfahren nach Anspruch 13, wobei der Reinigungsschritt ausgewählt ist aus der Gruppe bestehend aus Behandlung mit Aktivkohle, Ionenaustausch und Kristallisation.

15. Verfahren nach Anspruch 10, zudem umfassend die Isolierung von L-Lysin aus der Fermentationsbrühe und die Kristallisation von L-Lysin in Form eines Salzes.

## Revendications

1. Bactérie de l'espèce *Corynebacterium glutamicum* ayant la capacité à excréter de la L-lysine contenant dans son chromosome un polynucléotide codant pour un polypeptide ayant l'activité d'une malate:quinone oxydoréductase et comprenant la séquence d'acides aminés de SEQ ID NO : 2, dans laquelle l'acide aminé valine à la position 228 est substitué par un acide aminé protéinogène différent.

2. Bactérie selon la revendication 1, dans laquelle ledit acide aminé à la position 228 de la séquence d'acides aminés de SEQ ID NO : 2 est l'acide aspartique ou l'acide glutamique.

3. Bactérie selon les revendications 1 ou 2, dans laquelle ledit acide aminé à la position 228 de la séquence d'acides aminés de SEQ ID NO : 2 est l'acide aspartique.

4. Bactérie selon la revendication 3, dans laquelle le polynucléotide codant pour ladite séquence d'acides aminés comprend la séquence nucléotidique des positions 1001 à 2500 de SEQ ID NO : 1, la base azotée à la position 1683 étant l'adénine.

5. Bactérie selon la revendication 3, dans laquelle le polynucléotide codant pour ladite séquence d'acides aminés comprend la séquence nucléotidique des positions 1001 à 2500 de SEQ ID NO : 3.

6. Bactérie selon l'une quelconque des revendications 1 à 5, dans laquelle l'extrémité 5' du polynucléotide codant pour ladite séquence d'acides aminés est directement reliée à l'extrémité 3' du polynucléotide comprenant la séquence nucléotidique des positions 781 à 1000 de SEQ ID NO : 1 ou de SEQ ID NO : 3.

7. Bactérie selon la revendication 6, dans laquelle le polynucléotide codant pour ladite séquence d'acides aminés comprend la séquence nucléotidique des positions 781 à 2500 de SEQ ID NO : 3.

8. Bactérie selon la revendication 6, dans laquelle le polynucléotide codant pour ladite séquence d'acides aminés comprend la séquence nucléotidique des positions 781 à 2500 de SEQ ID NO : 1, la base azotée à la position 1683 étant l'adénine.

9. Procédé de production par fermentation d'une L-lysine comprenant les étapes de
a) culture de la bactérie telle que définie dans l'une quelconque des revendications 1 à 8 dans un milieu adapté dans des conditions adaptées, et
b) accumulation de L-lysine dans le milieu pour former un bouillon de fermentation contenant de la L-lysine.

10. Procédé selon la revendication 9, ledit procédé comprenant la fabrication d'un produit contenant de la L-lysine à partir dudit bouillon de fermentation.

11. Procédé selon l'une quelconque des revendications 9 à 10, ledit procédé comprenant l'extraction ou l'élimination substantielle de l'eau dudit bouillon de fermentation.

12. Procédé selon la revendication 11, dans lequel au moins 40 % (m/m), de préférence au moins 90 % (m/m), plus préférablement au moins 95 % (m/m) d'eau sont extraits du bouillon de fermentation.

13. Procédé selon la revendication 12, dans lequel ladite fabrication comprend une étape de purification.

14. Procédé selon la revendication 13, dans lequel ladite étape de purification est choisie dans le groupe constitué du traitement avec du charbon, de l'échange ionique et de la cristallisation.

15. Procédé selon la revendication 10, comprenant en outre l'isolement de L-lysine à partir du bouillon de fermentation et la cristallisation de L-lysine sous la forme d'un sel.
